# EUROPEAN PATENT SPECIFICATION

(11) **EP 2 684 050 B1**
(45) Date of publication and mention of the grant of the patent: **11.05.2016**
(21) Application number: 12707315.3
(22) Date of filing: 07.03.2012
(51) Int. Cl.: G01N 33/68

(54) **ARMET AS MARKER FOR CHRONIC OBSTRUCTIVE PULMONARY DISEASE (COPD)**
ARMET ALS MARKER FÜR DIE CHRONISCH OBSTRUKTIVE LUNGENERKRANKUNG (COPD)
ARMET EN TANT QUE MARQUEUR POUR UNE MALADIE PULMONAIRE OBSTRUCTIVE CHRONIQUE (COPD)

(30) Priority: 11.03.2011 EP 11157918
(43) Date of publication of application: 15.01.2014
(73) Proprietor: Roche Diagnostics GmbH, 68305 Mannheim (DE); F. Hoffmann-La Roche AG, 4070 Basel (CH)
(72) Inventor: KARL, Johann, 82380 Peissenberg (DE); RIEDLINGER, Julia, 85521 Ottobrunn (DE); ROESSLER, Markus, 82110 Germering (DE)
(74) Representative: Burger, Alexander
(86) International application number: PCT/EP2012/053843
(87) International publication number: WO 2012/123296

(56) References cited:
- WO-A2-2006/105252
- LINDHOLM P ET AL: "MANF is widely expressed in mammalian tissues and differently regulated after ischemic and epileptic insults in rodent brain", MOLECULAR AND CELLULAR NEUROSCIENCES, SAN DIEGO, US, vol. 39, no. 3, 29 October 2008 (2008-10-29), pages 356-371, XP025535501, ISSN: 1044-7431, DOI: 10.1016/J.MCN.2008.07.016 [retrieved on 2008-07-30]

## Description

### Field of the Invention

The present invention relates to an in vitro method aiding in the assessment of chronic obstructive pulmonary disease (= COPD). It discloses the use of the protein ARMET as a marker of COPD. Furthermore, it especially relates to a method for assessing COPD from a serum, plasma, or whole blood sample, derived from an individual by measuring the protein ARMET in said sample in vitro.

### Background of the Invention

Chronic obstructive pulmonary disease (COPD) is a disease characterised by chronic inflammation and irreversible airflow obstruction with a decline in the lung function parameter FEV1 that is more rapid than normal. This leads to a limitation of the flow of air to and from the lungs causing shortness of breath. The disease has two major aspects of pathology, namely chronic bronchitis, characterised by mucus hypersecretion from the conducting airways, and emphysema, characterised by destructive changes in the alveoli. In clinical practice, COPD is defined by its characteristically low airflow on lung function tests (Nathell, L., et al., Respiratory Research 8 (2007) 89). In contrast to asthma, this limitation is poorly reversible and usually gets progressively worse over time.

Worldwide, COPD ranked as the sixth leading cause of death in 1990. It is projected to be the fourth leading cause of death worldwide by 2030 due to an increase in smoking rates and demographic changes in many countries (Mathers, C.D., et al., PLoS Med. 3 (2006) e442). COPD is the 4th leading cause of death in the U.S., and the economic burden of COPD in the U.S. in 2007 was $42.6 billion in health care costs and lost productivity.

COPD is caused by noxious particles or gas, most commonly from tobacco smoking, which triggers an abnormal inflammatory response in the lung (Rabe, K.F., et al., Am. J. Respir. Crit. Care Med. 176 (2007) 532-555 and Hogg, J.C., et al., N. Engl. J. Med. 350 (2004) 2645-2653). The inflammatory response in the larger airways is known as chronic bronchitis, which is diagnosed clinically when people regularly cough up sputum. In the alveoli, the inflammatory response causes destruction of the tissues of the lung, a process known as emphysema. The natural course of COPD is characterized by occasional sudden worsenings of symptoms called acute exacerbations, most of which are caused by infections or air pollution.

Many of the symptoms of COPD are shared by other respiratory diseases such as asthma, bronchitis, pulmonary fibrosis and tuberculosis. The current gold standard for the diagnosis of COPD requires a lung function tests (spirometry), which is a time consuming and costly procedure which can be only realized by a specialized lung physician. A spirometry test, for example, is highly dependent on patient cooperation and effort, and is normally repeated at least three times to ensure reproducibility.

Chronic bronchitis can be diagnosed by asking the patient whether they have a "productive cough" i.e. one that yields sputum.

Asthma differs from COPD in its pathogenic and therapeutic response, and should therefore be considered a different clinical entity. However, some patients with asthma develop poorly reversible airflow limitation, which are currently indistinguishable from patients with COPD but for practical purposes are treated as asthma. The high prevalence of asthma and COPD in the general population results in the co-existence of both disease entities in many individuals. This is characterised by significant airflow limitation and a large response to bronchodilators. In these patients, the forced expiratory volume in one second (FEV1) does not return to normal and frequently worsens over time.

It is known that CRP serum levels are significantly higher in patients with asthma as compared to normal controls (Fujita, M., et al., Ann Allergy Asthma Immunol. 99 (2007) 48-53). CRP increases in response to a number of infectious and inflammatory conditions and therefore it is not specific to COPD (Donaldson, G.C., Am. J. Respir. Crit. Care Med. 175 (2007) 209-210). Therefore CRP does not meet the criteria regards diagnostic accuracy required for a COPD screening tool.

The current detection method of COPD, spirometry, seems to be not appropriate for use as a general screening tool. Spirometry is very costly, time consuming and not affordable to health care systems for a general and broad use in screenings of large numbers of subjects. Furthermore, its results strongly depend on patients' compliance.

It is described in the art that in COPD also an increase in neutrophils, macrophages and T-lymphocytes (specifically CD8+) in various parts of the lungs is observed, which relate to the degree of airflow limitation (Saetta, M., et al., Am. J. Respir. Crit. Care Med. 157 (1998) 822-826). There may be an increase in eosinophils in some patients, particularly during exacerbations (Saetta, M., et al., Am. J. Respir. Crit. Care Med. 150 (1994) 1646-1652 and Saetta, M., et al., Clin. Exp. Allergy 26 (1996) 766-774). These inflammatory cells are capable of releasing a variety of cytokines and inflammatory mediators, most notably leukotriene 4, interleukin-8 and TNF-α. This inflammatory pattern is markedly different from that seen in patients with bronchial asthma. Inflammatory changes may persist after quitting smoking. The mechanisms explaining the perpetuation of this inflammatory response in the absence of the inciting events are unknown.

Thus, it is an object of the present invention to provide a simple and cost-efficient procedure of COPD assessments, e.g. to identify individuals suspected of having COPD. For this purpose, a general COPD marker present in the circulation which is detectable in body fluids (e.g. blood, serum or plasma) has to be found.

In order to be of clinical utility, a new diagnostic marker as a single marker should be comparable to other markers known in the art, or better. Or, a new marker should lead to an improvement in diagnostic sensitivity or specificity either if used alone or if used in combination with one or more other markers, respectively. The diagnostic sensitivity or specificity of a test is best assessed by its receiver-operating characteristics, which will be described in detail below.

Whole blood, serum or plasma are the most widely used sources of sample in clinical routine. The identification of an early COPD marker that would aid in the reliable COPD detection or provide early prognostic information could lead to a method that would greatly aid in the diagnosis and in the management of this disease. Therefore, an urgent clinical need exists to improve the in vitro assessment of COPD. It is especially important to improve the early diagnosis of COPD, since for patients diagnosed in early stages of CODP the chances of reversibility of lung damages are much higher as compared to those patients diagnosed at a more progressed stage of disease.

There is a need in the art to identify a reliable and straightforward indicator of the COPD disease state (for example, a surrogate marker) both in order to reliably distinguish the symptoms of COPD from those of the above mentioned other respiratory diseases, to predict changes in disease severity, disease progression and response to medicine.

It was the object of the present invention to investigate whether a biochemical marker can be identified which may be used in assessing COPD in vitro. In particular, the inventors of the present invention investigated whether a biochemical marker could be identified for the assessment of COPD in a body fluid sample.

### Summary of the Invention

It has now been found that the use of protein ARMET can at least partially overcome some of the problems of the methods available for assessment of COPD presently known.

Surprisingly it was found in the present invention that an in vitro determination of the concentration of protein ARMET in a serum, plasma, or whole blood sample allows for the assessment of COPD. In this context it was found that an elevated concentration of said protein ARMET in such sample obtained from an individual compared to a reference concentration for protein ARMET is indicative for the presence of COPD.

Disclosed herein is an in vitro method for assessing COPD comprising determining in a serum, plasma, or whole blood sample the concentration of protein ARMET by an immunological detection method and using the determined result, particularly the concentration determined, in the assessment of COPD.

The invention also relates to an in vitro method for assessing chronic obstructive pulmonary disease (COPD) in a human subject, comprising a) determining the concentration of protein ARMET in a serum, plasma, or whole blood sample, and b) comparing the concentration of protein ARMET determined in step (a) with a reference concentration of protein ARMET, wherein a concentration of protein ARMET above a reference concentration is indicative for COPD.

In a further embodiment the present invention relates to the use of the protein ARMET in the in vitro assessment of COPD in a human serum, plasma, or whole blood sample, wherein a concentration of protein ARMET above a reference concentration for protein ARMET is indicative for COPD.

Further disclosed is the use of a marker panel comprising protein ARMET and protein NNMT in the in vitro assessment of COPD in a human serum, plasma, or whole blood sample, wherein a concentration of protein ARMET above a reference concentration for protein ARMET is indicative for COPD.

In a further embodiment the present invention relates to the use of the in vitro method for assessing COPD according to the present invention to differentiate COPD from other types of lung diseases, preferably asthma.

Additional aspects and advantages of the present invention will be apparent in view of the detailed description which follows.

### Detailed Description of the Invention

The inventors of the present invention have surprisingly been able to demonstrate that the marker protein ARMET is useful in the assessment of COPD. Due to the uncertainties of classifying the various stages of lung damage, and especially of COPD by state of the art methods, it may well be that the protein ARMET may become one of the pivotal criteria in the assessment of patients with COPD in the future.

The method of the present invention is suitable for the assessment of COPD. Increased concentrations of protein ARMET in a serum, plasma, or whole blood sample as compared to normal controls have been found to be indicative of COPD.

In one embodiment the present invention relates to an in vitro method for assessing chronic obstructive pulmonary disease (COPD) in a human subject, comprising a) determining the concentration of protein ARMET in a serum, plasma, or whole blood sample, and b) comparing the concentration of protein ARMET determined in step (a) with a reference concentration of protein ARMET, wherein a concentration of protein ARMET above a reference concentration is indicative for COPD.

**ASC**, the "apoptosis-associated speck-like protein containing a caspase-associated recruitment domain" is also known as "target of methylation-induced silencing 1" (TMS1) (Swiss-PROT: Q9ULZ3). The ASC protein in the sense of the present invention, characterized by the sequence given in SEQ ID NO: 1, is a 22 kDa protein. Caspase-associated recruitment domains (CARDs) mediate the interaction between adaptor proteins such as APAF1 (apoptotic protease activating factor 1) and the pro-form of caspases (e.g., CASP 9) participating in apoptosis. ASC is a member of the CARD-containing adaptor protein family. In WO 2006/105252 is has been shown, that the gene expression level of ASC (= CARD-9) is indicative for the diagnosis of COPD.

The biological role and function of **ARMET** (arginine-rich, mutated in early stage tumors, ARP, Swiss-PROT ID: P55145) protein remains largely elusive. The ARMET protein in the sense of the present invention, characterized by the sequence given in SEQ ID NO: 2, is a 20.3 kDa protein. The ARMET protein consists of 179 amino acids, and carries a predicted signal sequence (aa 1-21). The corresponding gene is located in chromosomal band 3p21.1 and was first characterized by Shridhar, V., et al., (Oncogene 12 (1996) 1931-1939). The gene is highly conserved and can be found many mammalian species, like rat, mouse, cow, and hamster. ARMET was named as such, because initial studies suggested ARMET to be 50 amino acids longer at the N-terminus carrying an arginine-rich region (Shridhar, V., et al., Oncogene 12 (1996) 1931-1939; Shridhar, R., et al., Cancer Res. 56 (1996) 5576-5578; Shridhar, V., et al., Oncogene 14 (1997) 2213-2216). However, more recent studies indicate transcribed evidence for a smaller open reading frame that does not encode the arginine tract (Tanaka, H., et al., Oncol. Rep. 7 (2000) 591-593; Mizobuchi, N., et al., Cell Struct. Funct. 32 (2007) 41-50). With the corresponding protein size correction, the initially described mutated codon (ATG50) is now identified to be the initiation codon. Petrova, P., et al., (J. Mol. Neurosci. 20 (2003) 173-188) purified the ARMET gene product from conditioned medium of a rat mesencephalic type-1 astrocyte cell line and named it MANF (Mensencephalic Astrocyte-dervied Neurotrophic Factor). Most recent studies demonstrated that ARMET is upregulated by the "unfolded protein response" (UPR), a process which is activated once misfolded proteins accumulate in the endoplasmatic reticulum (ER) (Tanaka, H., et al., Oncol. Rep. 7 (2000) 591-593; Apostolou, A., et al., Exp. Cell Res. 314 (2008) 2454-2467). Based on this study ARMET is characterized as a novel secreted mediator of the adaptive pathway of UPR.

The **NNMT** (nicotinamide N-methyltransferase; Swiss-PROT: P40261) protein in the sense of the present invention, characterized by the sequence given in SEQ ID NO: 3, is a 29.6 kDa protein and has an isoelectric point of 5.56. NNMT catalyzes the N-methylation of nicotinamide and other pyridines. This activity is important for biotransformation of many drugs and xenobiotic compounds. The protein has been reported to be predominantly expressed in liver and is located in the cytoplasm. NNMT has been cloned from cDNA from human liver and contained a 792-nucleotide open reading frame that encoded a 264-amino acid protein with a calculated molecular mass of 29.6 kDa. (Aksoy, S., et al., J. Biol. Chem. 269 (1994) 14835-14840). Little is known in the literature about a potential role of the enzyme in human COPD. In the Am. J. of Repiratory and Critical Care Medicine vol. 181, no. 8, 798-805 a higher mRNA expression of NNMT in skeletal muscle cells of COPD patients has been observed. In a study it has been shown that NNMT is a useful biomarker for lung cancer (LC) (J. of Cancer Res. and Clin. Onc. vol. 136, no. 9 (2009) 1223.1229). In said study it has been found that serum levels of NNMT were significantly higher in LC patients than in COPD patients and healthy donors.

Flap endonuclease-1 protein (= **FEN1,** FEN-1), Swiss-PROT ID: P39748 in the sense of the present invention, is a nuclear protein of 380 amino acids with a molecular weight of 42.6 kDa, characterized by the sequence given in SEQ ID NO: 4. The coding sequence of human FEN1 was predicted by Murray in 1994 (Murray, J.M., et al., Mol. Cell. Biol. 14 (1994) 4878-4888) from a newly cloned sequence. Based on the function of the yeast homolog rad2 a function in high fidelity chromosome segregation and in the repair of UV-induced DNA damage was suggested. As these are fundamental processes in chromosomal integrity, the authors also proposed an involvement of the protein in cancer avoidance. The gene locus on human chromosome 11 was later identified by Hiraoka, et al. (Hiraoka, L.R., et al., Genomics 25 (1995) 220-225) and Taylor, et al. (Taylor, T.D., et al., Nature 440 (2006) 497-500). The functions of FEN1 and its interactions with DNA have been the focus of numerous studies (Robins, P., et al., J. Biol. Chem. 269 (1994) 28535-28538); Shen, B., et al., J. Biol. Chem. 271 (1996) 9173-9176; Hasan, S., et al., Mol. Cell 7 (2001) 1221-1231; Qiu, J., et al., J. Biol. Chem. 277 (2002) 24659-24666 and Sakurai, S., et al, EMBO J. 24 (2005) 683-693). Several enzymatic functions in DNA metabolism have been demonstrated including endonuclease activity that cleaves the 5'-overhanging flap structure generated by displacement synthesis when DNA polymerase encounters the 5'-end of a downstream Okazaki fragment. Additionally FEN1 also possesses a 5' to 3' exonuclease activity on niked or gapped double-stranded DNA, and exhibits RNase H activity. These have been reviewed by Shen, et al. (Shen, B., et al., BioEssays 27 (2005) 717-729) or Liu, et al. (Liu, Y., et al., Annu. Rev. Biochem. 73 (2004) 589-615).

The AP endonuclease (**APEX1,** APEX-1) (Swiss-Prot. P27695) in the sense of the present invention is characterized by the sequence given in SEQ ID NO: 5. The unprocessed precursor molecule consists of 318 amino acids and has a molecular weight of 35.6 kDa. APEX1 is involved in DNA repair and excises the apurinic or apyrimidinic site of DNA strands. Such abasic sites are relative frequently generated either spontaneously or through chemical agents or by DNA glycosylases that remove specific abnormal bases.

AP sites are pre-mutagenic lesions that can prevent normal DNA replication so the cell contains systems to identify and repair such sites. (Barzilay, G. and Hickson, I.D., Bioessays 17 (1995) 713-719). The 3D structure was elucidated and the amino acids involved in endonuclease activity were identified (Barizilay, G., et al., Nature Structural Biology 2 (1995) 561-567; Gorman, M.A., et al., EMBO Journal 16 (1997) 6548-6558; Beernink, P., et al., J. Mol. Biol. 307 (2001) pp 1023-1034). APEX1 is also a redox regulator of various transcription factors such as c-Fos, c-Jun, NF-KB and HIF-1. This activity seems to be independent from the endonuclease activity. Both functions are located on different domains of the protein (Barzilay, G. and Hickson, I.D., Bioessays 17 (1995) 713-719). Phosphorylation of APEX1 by protein kinase C increases redox activity whereas the unphosphorylated form is involved in DNA-repair (Yacoub, A., et al., Cancer Res. 57 (1997) 5457-5459). One phosphorylation site, Y 261, (according to the Swissprot sequence) was identified by Rush, J., et al., (Nature Biotech, 23 (2005) 94-101).

**Seprase,** also known as fibroblast activation protein (= FAP), in the sense of the present invention is as a 170 kDa glycoprotein having gelatinase and dipeptidyl peptidase activity consisting of two identical monomeric Seprase units (Pineiro-Sanchez, M.L., et al., J. Biol. Chem. 272 (1997) 7595-7601; Park, J.E., et al., J. Biol. Chem. 274 (1999) 36505-36512). The monomer of the human membrane bound Seprase protein comprises 760 amino acids and is shown in SEQ ID NO: 6. Human Seprase is predicted to have its first 4 N-terminal residues within the fibroblast cytoplasm, followed by a 21-residue transmembrane domain and then a 734 residue extracellular C-terminal catalytic domain (Goldstein, L.A., et al., Biochim. Biophys. Acta. 1361 (1997) 11-19; Scanlan, M.J., et al., Proc. Natl. Acad. Sci. USA 91 (1994) 5657-5661). A shorter form of human Seprase protein is known to a person skilled in the art as soluble Seprase or circulating antiplasmin-cleaving enzyme (= APCE) (Lee, K.N., et al., Blood 103 (2004) 3783-3788; Lee, K.N., et al., Blood 107 (2006) 1397-1404), comprising the amino acid positions 26-760 from Swissprot database Accession number Q12884. The dimer of soluble Seprase is a 160 kDa glycoprotein consisting of two identical monomeric soluble Seprase protein units. Piñeiro-Sánchez et al. (supra) found that a increased expression of Seprase correlates with the invasive phenotype of human melanoma and carcinoma cells. Henry, L.R., et al., Clin. Cancer Res. 13 (2007) 1736-1741 describe that human colon tumor patients having high levels of stromal Seprase are more likely to have aggressive disease progression and potential development of metastases or recurrence.

Human dipeptidyl peptidase IV (= DPPIV), which is also known as CD26, is in the sense of the present invention a 110 kDa cell surface molecule. The amino acid sequence of human DPPIV protein comprises 766 amino acids and is shown in SEQ ID NO: 7 (Swissprot database Accession No. P27487). It contains intrinsic dipeptidyl peptidase IV activity which selectively removes N-terminal dipeptide from peptides with proline or alanine in the third amino acid position. It interacts with various extracellular molecules and is also involved in intracellular signal transduction cascades. The multifunctional activities of human DPPIV are dependent on cell type and intracellular or extracellular conditions that influence its role as a proteolytic enzyme, cell surface receptor, co-stimulatory interacting protein and signal transduction mediator. Human DPPIV has a short cytoplasmatic domain from amino acid position 1 to 6, a transmembrane region from amino acid position 7 to 28, and an extracellular domain from amino acid position 29 to 766 with intrinsic dipeptidyl peptidase IV (DPPIV) activity. Human soluble dipeptidyl peptidase IV (= soluble DPPIV) amino acid sequence comprises the amino acid positions 29 to 766 from Swissprot database Accession number P27487. The dimer of soluble DPPIV is a 170 kDa glycoprotein consisting of two identical monomeric soluble DPPIV units.

The "soluble DPPIV/Seprase protein complex" (= DPPIV/Seprase) in the sense of the present invention refers to the soluble complex formed of a soluble DPPIV homodimer (170 kDa) and a soluble Seprase homodimer (160 kDa) with a molecular weight of 330 kDa. Under certain conditions this complex may form a double complex having a molecular weight of 660 kDa.

As obvious to the skilled artisan, the present invention shall not be construed to be limited to the full-length protein ARMET of SEQ ID NO: 2. Physiological or artificial fragments of protein ARMET, secondary modifications of protein ARMET, as well as allelic variants of protein ARMET are also encompassed by the present invention. Variants of a polypeptide are encoded by the same gene, but may differ in their isoelectric point (=PI) or molecular weight (=MW), or both e.g., as a result of alternative mRNA or pre-mRNA processing. The amino acid sequence of a variant is to 95% or more identical to the corresponding marker sequence. Artificial fragments preferably encompass a peptide produced synthetically or by recombinant techniques, which at least comprises one epitope of diagnostic interest consisting of at least 6, 7, 8, 9 or 10 contiguous amino acids as derived from the sequence disclosed in SEQ ID NO: 2. Such fragment may advantageously be used for generation of antibodies or as a standard in an immunoassay.

It would appear that in the prior art the presence or level of the protein ARMET in a body fluid is not known to have a diagnostic utility in the assessment of COPD. The inventors of the present invention have now found and could establish that an increased concentration for protein ARMET as determined from a serum, plasma, or whole blood sample derived from an individual is indicative for COPD.

The practicing of the present invention will employ, unless otherwise indicated, conventional techniques of molecular biology (including recombinant techniques), microbiology, cell biology, biochemistry, and immunology, which are within the skill of the art. Such techniques are explained fully in the literature, such as, Sambrook, et al., Molecular Cloning: A Laboratory Manual, second edition (1989); Gait, M.J., (ed.), Oligonucleotide Synthesis (1984); Freshney, R.I., (ed.), Animal Cell Culture (1987); Methods in Enzymology (Academic Press, Inc.); Ausubel, F.M., et al., (eds.) Current Protocols in Molecular Biology (1987), and periodic updates); Mullis, et al., (eds.), PCR: The Polymerase Chain Reaction (1994).

Unless defined otherwise, technical and scientific terms used herein have the same meaning as commonly understood by one of ordinary skill in the art to which this invention belongs. Singleton, et al., Dictionary of Microbiology and Molecular Biology, 2nd ed., John Wiley & Sons, New York, N.Y. (1994); March, Advanced Organic Chemistry Reactions, Mechanisms and Structure, 4th ed., John Wiley & Sons (New York, N.Y. 1992); Lewin, B., Genes V, published by Oxford University Press (1994), ISBN 0-19-854287 9); Kendrew, J., et al. (eds.), The Encyclopedia of Molecular Biology, published by Blackwell Science Ltd. (1994), ISBN 0-632-02182-9); and Meyers, R.A., (ed.), Molecular Biology and Biotechnology: a Comprehensive Desk Reference, published by VCH Publishers, Inc. (1995), ISBN 1-56081-569 8) provide one skilled in the art with a general guide to many of the terms used in the present application.

As used herein, each of the following terms has the meaning associated with it in this section.

The articles "a" and "an" are used herein to refer to one or to more than one (i.e. to at least one) of the grammatical object of the article. By way of example, "a marker" means one marker or more than one marker. The term "at least" is used to indicate that optionally one or more than one further objects may be present.

The expression "one or more" denotes 1 to 50, preferably 1 to 20 also preferred 2, 3, 4, 5, 6, 7, 8, 9, 10, 12, or 15.

The term "marker" or "biochemical marker" as used herein refers to a molecule to be used as a target for analyzing an individual's test sample. In one embodiment examples of such molecular targets are proteins or polypeptides. Proteins or polypeptides used as a marker in the present invention are contemplated to include naturally occurring variants of said protein as well as fragments of said protein or said variant, in particular, immunologically detectable fragments. Immunologically detectable fragments preferably comprise at least 6, 7, 8, 10, 12, 15 or 20 contiguous amino acids of said marker polypeptide. One of skill in the art would recognize that proteins which are released by cells or present in the extracellular matrix may be damaged, e.g., during inflammation, and could become degraded or cleaved into such fragments. Certain markers are synthesized in an inactive form, which may be subsequently activated by proteolysis. As the skilled artisan will appreciate, proteins or fragments thereof may also be present as part of a complex. Such complex also may be used as a marker in the sense of the present invention. In addition, or in the alternative a marker polypeptide or a variant thereof may carry a post-translational modification. Preferred posttranslational modifications are glycosylation, acylation, or phosphorylation.

The term "label" as used herein refers to any substance that is capable of producing a signal via direct or indirect detection.

For direct detection the labeling group or label suitable for use in the present invention can be selected from any known detectable marker groups, but are not limited to, chromogens, fluorescent, chemiluminescent groups (e.g. acridinium esters or dioxetanes), electrochemiluminescent compounds, catalysts, enzymes, enzymatic substrates, dyes, fluorescent dyes (e.g. fluorescein, coumarin, rhodamine, oxazine, resorufin, cyanine and derivatives thereof), colloidal metallic and nonmetallic particles, and organic polymer latex particles. Other examples of labeling groups are luminescent metal complexes, such as ruthenium or europium complexes, enzymes, e.g. as used for ELISA, and radioisotopes.

Indirect detection systems comprise, for example, that the detection reagent, e.g. the detection antibody, is labeled with a first partner of a bioaffine binding pair. Examples of suitable binding pairs are hapten or antigen/antibody, biotin or biotin analogues such as aminobiotin, iminobiotin or desthiobiotin/avidin or streptavidin, sugar/lectin, nucleic acid or nucleic acid analogue/complementary nucleic acid, and receptor/ligand, e.g. steroid hormone receptor/steroid hormone. Preferred first binding pair members comprise hapten, antigen and hormone. Especially preferred are haptens like digoxin and biotin and analogues thereof. The second partner of such binding pair, e.g. an antibody, streptavidin, etc., usually is labeled to allow for direct detection, e.g. by the labels as mentioned above.

The term "assessing chronic obstructive pulmonary disease" or "assessing COPD" is used to indicate that the method according to the present invention will alone or together with other markers or variables, e.g., aid the physician to establish or confirm the absence or presence of COPD. The method will e.g. be useful to establish or confirm the absence or presence of COPD.

A "marker for COPD" in the sense of the present invention is a marker that, as single marker, or if combined with the marker ARMET, adds relevant information in the assessment of COPD to the diagnostic question under investigation. The information is considered relevant or of additive value if at a given specificity the sensitivity, or if at a given sensitivity the specificity, respectively, for the assessment of COPD can be improved by including said marker into a marker panel (marker combination) already comprising the marker ARMET. Preferably the improvement in sensitivity or specificity, respectively, is statistically significant at a level of significance of p = 0.05, 0.02, 0.01 or lower.

The term "sample" or "test sample" as used herein refers to a biological sample obtained from an individual for the purpose of evaluation in vitro. In the methods of the present invention, the sample or patient sample may comprise in an embodiment of the present invention any body fluid. Preferred samples are body fluids, such as serum, plasma, or whole blood, with serum or plasma being most preferred.

Protein ARMET, particularly soluble forms of protein ARMET, are determined in vitro in an appropriate sample. Preferably, the sample is derived from a human subject, e.g. a COPD patient or a person in risk of COPD or a person suspected of having COPD. Also preferred protein ARMET is determined in a serum or plasma sample.

The term "reference sample" as used herein refers to a biological sample provided from a reference group of apparently healthy individuals for the purpose of evaluation in vitro. The term "reference concentration" as used herein refers to a value established in a reference group of apparently healthy individuals.

It is known to a person skilled in the art that the measurement results of step (a) according to the method(s) of the present invention will be compared to a reference concentration. Such reference concentration can be determined using a negative reference sample, a positive reference sample, or a mixed reference sample comprising one or more than one of these types of controls. A negative reference sample preferably will comprise a sample from non smokers, control smokers with no diagnosis of COPD, asthma or various combinations thereof. A positive reference sample preferably will comprise a sample from a subject with the diagnosis of COPD.

The expression "comparing the concentration determined to a reference concentration" is merely used to further illustrate what is obvious to the skilled artisan anyway. A reference concentration is established in a control sample. The control sample may be an internal or an external control sample. In one embodiment an internal control sample is used, i.e. the marker level(s) is(are) assessed in the test sample as well as in one or more other sample(s) taken from the same subject to determine if there are any changes in the level(s) of said marker(s). In another embodiment an external control sample is used. For an external control sample the presence or amount of a marker in a sample derived from the individual is compared to its presence or amount in an individual known to suffer from, or known to be at risk of, a given condition; or an individual known to be free of a given condition, i.e., "normal individual". For example, a marker level in a patient sample can be compared to a level known to be associated with a specific course of COPD. Usually the sample's marker level is directly or indirectly correlated with a diagnosis and the marker level is e.g. used to determine whether an individual is at risk for COPD. Alternatively, the sample's marker level can e.g. be compared to a marker level known to be associated with a response to therapy in COPD patients, the diagnosis of COPD, the guidance for selecting an appropriate drug to COPD, in judging the risk of disease progression, or in the follow-up of COPD patients. Depending on the intended diagnostic use an appropriate control sample is chosen and a control or reference value for the marker established therein. It will be appreciated by the skilled artisan that such control sample in one embodiment is obtained from a reference population that is age-matched and free of confounding diseases. As also clear to the skilled artisan, the absolute marker values established in a control sample will be dependent on the assay used. Preferably samples from 100 well-characterized individuals from the appropriate reference population are used to establish a control (reference) value. Also preferred the reference population may be chosen to consist of 20, 30, 50, 200, 500 or 1000 individuals. Healthy individuals represent a preferred reference population for establishing a control value.

The term "measurement", "measuring" or "determining" preferably comprises a qualitative, a semi-quanitative or a quantitative measurement. In the present invention protein ARMET is measured in a body fluid sample. In a preferred embodiment the measurement is a semi-quantitative measurement, i.e. it is determined whether the concentration of protein ARMET is above or below a cut-off value. As the skilled artisan will appreciate, in a Yes- (presence) or No-(absence) assay, the assay sensitivity is usually set to match the cut-off value.

The values for protein ARMET as determined in a control group or a control population are for example used to establish a cut-off value or a reference range. A value above such cut-off value or out-side the reference range at its higher end is considered as elevated or as indicative for the presence of COPD.

In an embodiment a fixed cut-off value is established. Such cut-off value is chosen to match the diagnostic question of interest.

In an embodiment the cut-off is set to result in a specificity of 90%, also preferred the cut-off is set to result in a specificity of 95%, or also preferred the cut-off is set to result in a specificity of 98%.

In an embodiment the cut-off is set to result in a sensitivity of 90%, also preferred the cut-off is set to result in a sensitivity of 95%, or also preferred the cut-off is set to result in a sensitivity of 98%.

In one embodiment values for protein ARMET as determined in a control group or a control population are used to establish a reference range. In a preferred embodiment an concentration of protein ARMET is considered as elevated if the value determined is above the 90%-percentile of the reference range. In further preferred embodiments a concentration of protein ARMET is considered as elevated if the value determined is above the 95%-percentile, the 96%-percentile, the 97%-percentile or the 97.5%-percentile of the reference range.

A value above the cut-off value can for example be indicative for the presence of COPD. A value below the cut-off value can for example be indicative for the absence of COPD.

In a further preferred embodiment the measurement of protein ARMET is a quantitative measurement. In further embodiments the concentration of protein ARMET is correlated to an underlying diagnostic question.

A sample provided from a patient with already confirmed COPD in certain settings might be used as a positive control sample and preferably assayed in parallel with the sample to be investigated. In such setting a positive result for the marker protein ARMET in the positive control sample indicates that the testing procedure has worked on the technical level.

As the skilled artisan will appreciate, any such assessment is made in vitro. The sample (test sample) is discarded afterwards. The sample is solely used for the in vitro diagnostic method of the invention and the material of the sample is not transferred back into the patient's body. Typically, the sample is a body fluid sample, e.g., serum, plasma, or whole blood.

The method according to the present invention is based on a liquid or body fluid sample which is obtained from an individual and on the in vitro determination of protein ARMET in such sample. An "individual" as used herein refers to a single human or non-human organism. Thus, the methods and compositions described herein are applicable to both human and veterinary disease. Preferably the individual, subject, or patient is a human being.

Preferably the marker protein ARMET is specifically determined in vitro from a liquid sample by use of a specific binding agent.

In a preferred embodiment according to the present invention, the concentration of protein ARMET is determined. In an embodiment, the concentration of marker protein ARMET is specifically determined in vitro from a sample by use of a specific binding agent.

A specific binding agent is, e.g., a receptor for the protein ARMET, a lectin binding to protein ARMET, an antibody to protein ARMET, peptidebodies to protein ARMET, bispecific dual binders or bispecific antibody formats. A specific binding agent has at least an affinity of 10⁷ l/mol for its corresponding target molecule. The specific binding agent preferably has an affinity of 10⁸ l/mol or also preferred of 10⁹ l/mol for its target molecule.

As the skilled artisan will appreciate the term specific is used to indicate that other biomolecules present in the sample do not significantly bind to the binding agent specific for the protein ARMET sequence of SEQ ID NO: 2. Preferably, the level of binding to a biomolecule other than the target molecule results in a binding affinity which is at most only 10% or less, only 5% or less only 2% or less or only 1% or less of the affinity to the target molecule, respectively. A preferred specific binding agent will fulfill both the above minimum criteria for affinity as well as for specificity.

Examples of specific binding agents are peptides, peptide mimetics, aptamers, spiegelmers, darpins, ankyrin repeat proteins, Kunitz type domains, antibodies, single domain antibodies, (see: Hey, T., et al., Trends Biotechnol 23(10) (2005) 514-522) and monovalent fragments of antibodies.

In certain preferred embodiments the specific binding agent is a polypeptide.

In certain preferred embodiments the specific binding agent is an antibody or a monovalent antibody fragment, preferably a monovalent fragment derived from a monoclonal antibody.

Monovalent antibody fragments include, but are not limited to Fab, Fab'-SH, single domain antibody, Fv, and scFv fragments, as provided below.

The term "antibody" herein is used in the broadest sense and specifically covers monoclonal antibodies, polyclonal antibodies, multispecific antibodies (e.g. bispecific antibodies) formed from at least two intact antibodies, and antibody fragments so long as they exhibit the desired biological activity. In certain preferred embodiments the specific binding agent is an antibody or a monovalent antibody fragment, preferably a monovalent fragment derived from a monoclonal antibody.

An "isolated" antibody is one which has been identified and separated and/or recovered from a component of its natural environment. Contaminant components of its natural environment are materials which would interfere with research, diagnostic or therapeutic uses for the antibody, and may include enzymes, hormones, and other proteinaceous or nonproteinaceous solutes. In some embodiments, an antibody is purified (1) to greater than 95% by weight of antibody as determined by, for example, the Lowry method, and in some embodiments, to greater than 99% by weight; (2) to a degree sufficient to obtain at least 15 residues of N-terminal or internal amino acid sequence by use of, for example, a spinning cup sequenator, or (3) to homogeneity by SDS-PAGE under reducing or nonreducing conditions using, for example, Coomassie blue or silver stain. Isolated antibody includes the antibody in situ within recombinant cells since at least one component of the antibody's natural environment will not be present. Ordinarily, however, isolated antibody will be prepared by at least one purification step.

"Native antibodies" are usually heterotetrameric glycoproteins of about 150,000 daltons, composed of two identical light (L) chains and two identical heavy (H) chains. Each light chain is linked to a heavy chain by one covalent disulfide bond, while the number of disulfide linkages varies among the heavy chains of different immunoglobulin isotypes. Each heavy and light chain also has regularly spaced intrachain disulfide bridges. Each heavy chain has at one end a variable domain (VH) followed by a number of constant domains. Each light chain has a variable domain at one end (VL) and a constant domain at its other end; the constant domain of the light chain is aligned with the first constant domain of the heavy chain, and the light-chain variable domain is aligned with the variable domain of the heavy chain. Particular amino acid residues are believed to form an interface between the light-chain and heavy-chain variable domains.

The "variable region" or "variable domain" of an antibody refers to the amino-terminal domains of the heavy or light chain of the antibody. The variable domain of the heavy chain may be referred to as "VH." The variable domain of the light chain may be referred to as "VL." These domains are generally the most variable parts of an antibody and contain the antigen-binding sites.

The term "variable" refers to the fact that certain portions of the variable domains differ extensively in sequence among antibodies and are used in the binding and specificity of each particular antibody for its particular antigen. However, the variability is not evenly distributed throughout the variable domains of antibodies. It is concentrated in three segments called hypervariable regions (HVRs) both in the light-chain and the heavy-chain variable domains. The more highly conserved portions of variable domains are called the framework regions (FR). The variable domains of native heavy and light chains each comprise four FR regions, largely adopting a beta-sheet configuration, connected by three HVRs, which form loops connecting, and in some cases forming part of, the beta-sheet structure. The HVRs in each chain are held together in close proximity by the FR regions and, with the HVRs from the other chain, contribute to the formation of the antigen-binding site of antibodies (see Kabat, et al., Sequences of Proteins of Immunological Interest, 5th ed., National Institute of Health, Bethesda, MD (1991)). The constant domains are not involved directly in the binding of an antibody to an antigen, but exhibit various effector functions, such as participation of the antibody in antibody-dependent cellular toxicity.

The "light chains" of antibodies (immunoglobulins) from any vertebrate species can be assigned to one of two clearly distinct types, called kappa (κ) and lambda (λ), based on the amino acid sequences of their constant domains.

Depending on the amino acid sequences of the constant domains of their heavy chains, antibodies (immunoglobulins) can be assigned to different classes. There are five major classes of immunoglobulins: IgA, IgD, IgE, IgG, and IgM, and several of these may be further divided into subclasses (isotypes), e.g., IgG1, IgG2, IgG3, IgG4, IgA1, and IgA2. The subunit structures and three-dimensional configurations of different classes of immunoglobulins are well known and described generally in, for example, Abbas, et al., Cellular and Mol. Immunology, 4th ed., W.B. Saunders, Co. (2000). An antibody may be part of a larger fusion molecule, formed by covalent or non-covalent association of the antibody with one or more other proteins or peptides.

The terms "full-length antibody," "intact antibody," and "whole antibody" are used herein interchangeably to refer to an antibody in its substantially intact form, not antibody fragments as defined below. The terms particularly refer to an antibody with heavy chains that contain an Fc region.

"Antibody fragments" comprise a portion of an intact antibody, preferably comprising the antigen-binding region thereof. Examples of antibody fragments include Fab, Fab', F(ab')2, and Fv fragments; diabodies; linear antibodies; single-chain antibody molecules; and multispecific antibodies formed from antibody fragments.

Papain digestion of antibodies produces two identical antigen-binding fragments, called "Fab" fragments, each with a single antigen-binding site, and a residual "Fc" fragment, whose name reflects its ability to crystallize readily. Pepsin treatment yields a F(ab')2 fragment that has two antigen-combining sites and is still capable of cross-linking antigen.

"Fv" is the minimum antibody fragment which contains a complete antigen-binding site. In one embodiment, a two-chain Fv species consists of a dimer of one heavy- and one light-chain variable domain in tight, non-covalent association. In a single-chain Fv (scFv) species, one heavy- and one light-chain variable domain can be covalently linked by a flexible peptide linker such that the light and heavy chains can associate in a "dimeric" structure analogous to that in a two-chain Fv species. It is in this configuration that the three HVRs of each variable domain interact to define an antigen-binding site on the surface of the VH-VL dimer. Collectively, the six HVRs confer antigen-binding specificity to the antibody. However, even a single variable domain (or half of an Fv comprising only three HVRs specific for an antigen) has the ability to recognize and bind antigen, although at a lower affinity than the entire binding site.

The Fab fragment contains the heavy- and light-chain variable domains and also contains the constant domain of the light chain and the first constant domain (CH1) of the heavy chain. Fab' fragments differ from Fab fragments by the addition of a few residues at the carboxy terminus of the heavy chain CH1 domain including one or more cysteines from the antibody-hinge region. Fab'-SH is the designation herein for Fab' in which the cysteine residue(s) of the constant domains bear a free thiol group. F(ab')2 antibody fragments originally were produced as pairs of Fab' fragments which have hinge cysteines between them. Other chemical couplings of antibody fragments are also known.

"Single-chain Fv" or "scFv" antibody fragments comprise the VH and VL domains of an antibody, wherein these domains are present in a single polypeptide chain. Generally, the scFv polypeptide further comprises a polypeptide linker between the VH and VL domains that enables the scFv to form the desired structure for antigen binding. For a review of scFv, see, e.g., Pluckthuen, In: The Pharmacology of Monoclonal Antibodies, vol. 113, Rosenburg and Moore (eds.), Springer-Verlag, New York (1994) pp. 269-315.

The term "diabodies" refers to antibody fragments with two antigen-binding sites, which fragments comprise a heavy-chain variable domain (VH) connected to a light-chain variable domain (VL) in the same polypeptide chain (VH-VL). By using a linker that is too short to allow pairing between the two domains on the same chain, the domains are forced to pair with the complementary domains of another chain and create two antigen-binding sites. Diabodies may be bivalent or bispecific. Diabodies are described more fully in, for example, EP 0404 097; WO 1993/01161; Hudson, et al., Nat. Med. 9 (2003) 129-134; and Hollinger, et al., PNAS USA 90 (1993) 6444-6448. Triabodies and tetrabodies are also described in Hudson, et al., Nat. Med. 9 (2003) 129-134.

The term "monoclonal antibody" as used herein refers to an antibody obtained from a population of substantially homogeneous antibodies, i.e., the individual antibodies comprising the population are identical except for possible mutations, e.g., naturally occurring mutations, that may be present in minor amounts. Thus, the modifier "monoclonal" indicates the character of the antibody as not being a mixture of discrete antibodies. In certain embodiments, such a monoclonal antibody typically includes an antibody comprising a polypeptide sequence that binds a target, wherein the target-binding polypeptide sequence was obtained by a process that includes the selection of a single target binding polypeptide sequence from a plurality of polypeptide sequences. For example, the selection process can be the selection of a unique clone from a plurality of clones, such as a pool of hybridoma clones, phage clones, or recombinant DNA clones. It should be understood that a selected target binding sequence can be further altered, for example, to improve affinity for the target, to humanize the target-binding sequence, to improve its production in cell culture, to reduce its immunogenicity in vivo, to create a multispecific antibody, etc., and that an antibody comprising the altered target binding sequence is also a monoclonal antibody of this invention. In contrast to polyclonal antibody preparations, which typically include different antibodies directed against different determinants (epitopes), each monoclonal antibody of a monoclonal-antibody preparation is directed against a single determinant on an antigen. In addition to their specificity, monoclonal-antibody preparations are advantageous in that they are typically uncontaminated by other immunoglobulins.

A specific binding agent preferably is an antibody reactive with SEQ ID NO: 2.

For the achievements as disclosed in the present invention antibodies from various sources may be used. Standard protocols for obtaining antibodies can be as well used as modern alternative methods. Alternative methods for generation of antibodies comprise amongst others the use of synthetic or recombinant peptides, representing a clinically relevant epitope of ARMET for immunization. Alternatively, DNA immunization also known as DNA vaccination may be used. Clearly monoclonal antibodies or polyclonal antibodies from different species, e.g., rabbits, sheep, goats, rats or guinea pigs can be used. Since monoclonal antibodies can be produced in any amount required with constant properties, they represent ideal tools in development of an assay for clinical routine.

As the skilled artisan will appreciate now, that protein ARMET has been identified as a marker which is useful in the assessment of COPD. Various immunodiagnostic procedures may be used to reach data comparable to the achievements of the present invention.

For determination of protein ARMET the sample obtained from an individual is incubated in vitro with the specific binding agent for ARMET under conditions appropriate for formation of a binding agent ARMET complex. Such conditions need not be specified, since the skilled artisan without any inventive effort can easily identify such appropriate incubation conditions. The amount of binding agent ARMET complex is determined and used in the assessment of COPD. As the skilled artisan will appreciate there are numerous methods to determine the amount of the specific binding agent ARMET complex all described in detail in relevant textbooks (cf., e.g., Tijssen, P., supra, or Diamandis, E.P., and Christopoulos, T.K. (eds.), Immunoassay, Academic Press, Boston (1996)).

Immunoassays are well known to the skilled artisan. Methods for carrying out such assays as well as practical applications and procedures are summarized in related textbooks. Examples of related textbooks are Tijssen, P., Preparation of enzyme-antibody or other enzyme-macromolecule conjugates, In: Practice and theory of enzyme immunoassays, pp. 221-278, Burdon, R.H., and v. Knippenberg, P.H. (eds.), Elsevier, Amsterdam (1990), and various volumes of Colowick, S.P., and Caplan, N.O. (eds.), Methods in Enzymology, Academic Press, dealing with immunological detection methods, especially volumes 70, 73, 74, 84, 92 and 121.

The present invention also relates in an embodiment to the use of an antibody specifically binding to protein ARMET in a method according to the present invention.

In one embodiment in a method according to the present invention protein ARMET is measured in an immunoassay procedure.

In a further embodiment protein ARMET is detected in an enzyme-linked immunoassay (ELISA).

In a further embodiment protein ARMET is detected in a sandwich assay (sandwich-type assay format). In such assay, a first specific binding agent is used to capture protein ARMET on the one side and a second specific binding agent, which is labelled to be directly or indirectly detectable, is used on the other side. The specific binding agents used in a sandwich-type assay format may be antibodies specifically directed against protein ARMET. On the one hand, the detection may be carried out by using different capturing and labelled antibodies, i.e. antibodies which recognize different epitopes on the ARMET polypeptide. On the other hand, a sandwich-type assay may also be carried out with a capture and labelling antibody which is directed against the same epitope of protein ARMET. In this embodiment, only di- and multimeric forms of protein ARMET may be detected. In an embodiment an antibody to protein ARMET is used in a qualitative (ARMET present or absent) or quantitative (amount of ARMET is determined) immunoassay.

In a further embodiment the method according to the present invention is based on the measurement of ARMET, wherein said measurement of ARMET is performed in a sandwich immunoassay employing at least two antibodies reactive with at least two non-overlapping epitopes.

In a further embodiment protein ARMET is detected in a competitive assay. In such assay format a binding agent specifically binding to ARMET of SEQ ID NO: 2 is used. In a mixture labeled ARMET that has been added to the mixture and ARMET comprised in a sample compete for binding to the specific binding agent. The extent of such competition can be measured according to standard procedures.

The concentration of the protein ARMET in test samples may be determined in vitro using a specific ELISA, as already described above. Using this assay format, the inventors have shown that samples from patients already diagnosed as having COPD by classical methods, e.g. spirometry, can be distinguished from samples from apparently healthy individuals. Results are shown in the example section of this application.

The inventors of the present invention surprisingly are able to detect protein ARMET in a serum, plasma, or whole blood sample. Even more surprising they are able to demonstrate that the presence of protein ARMET in such serum, plasma, or whole blood sample obtained from an individual can be correlated to COPD. No tissue and no biopsy sample is required to make use of the marker ARMET in the assessment of COPD. Measuring the level of protein ARMET in (e.g. a small aliquot of) a simple serum, plasma, or whole blood sample is considered very advantageous in the field of COPD.

In a preferred embodiment the method according to the present invention is practiced with serum as sample material. In a further preferred embodiment the method according to the present invention is practiced with plasma as sample material. In a further preferred embodiment the method according to the present invention is practiced with whole blood as sample material.

In a further preferred embodiment, the present invention relates to use of protein ARMET as a marker molecule in the in vitro assessment of COPD from a serum, plasma, or whole blood obtained from an individual.

The ideal scenario for diagnosis would be a situation wherein a single event or process would cause the respective disease as, e.g., in infectious diseases. In all other cases correct diagnosis can be very difficult, especially when the etiology of the disease is not fully understood as is the case for COPD. As the skilled artisan will appreciate, no biochemical marker is diagnostic with 100% specificity and at the same time 100% sensitivity for a given multifactorial disease, as for example for COPD. Rather, biochemical markers are used to assess with a certain likelihood or predictive value an underlying diagnostic question, e.g., the presence, absence, or the severity of a disease. Therefore in routine clinical diagnosis, generally various clinical symptoms and biological markers are considered together in the assessment of an underlying disease. The skilled artisan is fully familiar with the mathematical/statistical methods that routinely are used to calculate a relative risk or likelihood for the diagnostic question to be assessed. In routine clinical practice various clinical symptoms and biological markers are generally considered together by a physician in the diagnosis, treatment, and management of the underlying disease.

COPD patients are traditionally treated with bronchodilators or steroids and examined by spirometry for reversibility of airflow obstruction. If reversibility is less than 15%, and particularly if they have a long history of smoking, then they would be classified as COPD patients.

The ATS (American Thoracic Society) criteria for diagnosing COPD are as follows:
- FEV1/FVC ratio < 0.7
- FEV < 70% predicted, < 15% reversibility to inhaled B2 agonist:
- 2 week oral prednisolone trial-less than 15% reversibility in FEV1
- Smoking history

FEV1 is the volume of air expelled from the lungs in one second, starting from a position of maximum inspiration and with the subject making maximum effort. FEV1% is the FEV1 expressed as a percentage of the forced vital capacity (FVC). The FVC is the total volume of air expelled from the lungs from a position of maximum inspiration with the subject making maximum effort. FEV1 may be measured using a spirometer to measure the volume of air expired in the first second of exhalation.

The spirometric classification of COPD according to the ATS (American Thoracic Society)/European respiratory Society 2004 is shown in Table 1. ATS COPD Stage 0 is currently no longer used in the ATS classification system.

**Table 1:**

| COPD Stage | Severity | Postbronchdilator FEV1/FVC | FEV 1% pred |
|---|---|---|---|
| 0 | At risk # | > 0.7 | ≥ 80% |
| I | Mild COPD | ≤ 0.7 | ≥ 80% |
| II | Moderate COPD | ≤ 0.7 | 50% - 80% |
| III | Severe COPD | ≤ 0.7 | 30% - 50% |
| IV | Very severe COPD | ≤ 0.7 | < 30% |

| | | | |
|---|---|---|---|
| FEV1: forced expiratory volume in one second; FVC: forced vital capacity; ^{#}: patients who smoke or have exposure to pollutants, have cough, sputum or dyspnoea, have family history of respiratory disease. | | | |

In the assessment of COPD the marker protein ARMET will be of advantage in one or more of the following aspects: assessment; screening; staging of disease; monitoring of disease progression; prognosis; guidance of therapy and monitoring of the response to therapy.

Preferred areas of diagnostic relevance in assessing an individual suspected or known to have COPD are screening, staging of disease, monitoring of disease progression and monitoring of the response to therapy.

### Screening (assessment whether individuals are at risk for developing COPD or have COPD):

Screening is defined as the systematic application of a test to identify individuals e.g. at risk individuals, for indicators of a disease, e.g., the presence of COPD. Preferably the screening population is composed of individuals known to be at higher than average risk of COPD. For example, a screening population for COPD is composed of individuals known to be at higher than average risk of COPD, like smokers and ex-smokers.

Screening in the sense of the present invention relates to the unbiased assessment of individuals regarding their risk for developing COPD. In an embodiment the method according to the present invention is used for screening purposes. I.e., it is used to assess human subjects without a prior diagnosis of COPD by a) determining the concentration of protein ARMET in a serum, plasma, or whole blood sample in vitro, and b) comparing the concentration of protein ARMET determined in step (a) with a reference concentration of protein ARMET, wherein a concentration of protein ARMET above the reference concentration is indicative for the presence of COPD.

Measurement of protein ARMET will aid the physician to assess the presence or absence of COPD in an individual suspected to have COPD.

In an embodiment the present invention relates to an in vitro method for assessing the presence or absence of chronic obstructive pulmonary disease (COPD) in a human subject, comprising a) determining the concentration of protein ARMET in a serum, plasma, or whole blood sample, and b) comparing the concentration of protein ARMET determined in step (a) with a reference concentration of protein ARMET, wherein a concentration of protein ARMET above the reference concentration is indicative for the presence of COPD.

In an embodiment the present invention relates to an in vitro method for assessing the presence or absence of chronic obstructive pulmonary disease (COPD) in a human subject, comprising a) determining the concentration of protein ARMET in a serum, plasma, or whole blood sample, b) comparing the concentration of protein ARMET determined in step (a) with a reference concentration of protein ARMET, and c) assessing the presence or absence of COPD based on the comparison of step (b), wherein a concentration of protein ARMET above the reference concentration is indicative for the presence of COPD.

In an embodiment the present invention relates to an in vitro method of assessing for a human subject the presence or absence of COPD, the method comprising a) determining the concentration of protein ARMET in a serum, plasma, or whole blood sample, and b) comparing the concentration of protein ARMET determined in step (a) with a cut-off value for protein ARMET established in a reference population, wherein a concentration of protein ARMET above the cut-off value is indicative for the presence of COPD. In an embodiment the present invention relates to an in vitro method of assessing for a human subject the presence or absence of COPD, the method comprising a) determining the concentration of protein ARMET in a serum, plasma, or whole blood sample, and b) comparing the concentration of protein ARMET determined in step (a) with a cut-off value for protein ARMET established in a reference population, wherein a concentration of protein ARMET below the cut-off value is indicative for the absence of COPD.

In a further embodiment the present invention relates to the use of the protein ARMET in the in vitro assessment of COPD in a serum, plasma, or whole blood sample, wherein a concentration of protein ARMET above a reference concentration for protein ARMET is indicative for COPD.

In a preferred embodiment said sample according the use is selected from the group consisting of serum, plasma and whole blood.

In a further embodiment the present invention relates to the use of the protein ARMET in the in vitro assessment of COPD in a serum, plasma, or whole blood sample, wherein a concentration of protein ARMET above a reference concentration for protein ARMET in a serum, plasma, or whole blood sample is indicative for the presence of COPD.

One embodiment of the present invention refers to the screening of a population to distinguish between individuals which are probably free from COPD and individuals which probably have COPD. The latter group of individuals may then be subject to further diagnostic procedures, e.g. by lung function testing, spirometry or other suitable means.

In an embodiment the in vitro method according to the present invention is characterized in that the assessment of the protein ARMET takes place for classifying a patient according to be at risk to have COPD for clinical decisions, particularly further treatment by means of medications for the treatment or therapy of COPD, and for treatment or therapy of infection/inflammatory diseases of the airway and lung, as well as for therapy control of an antibiotic treatment or therapeutic antibody treatment.

In an embodiment the present invention relates to an in vitro method for assessing whether an individual is at risk for developing COPD comprising the steps of a) determining the concentration of protein ARMET in a serum, plasma, or whole blood sample, and b) of assessing said individual's risk for developing COPD by comparing the concentration of protein ARMET determined in step (a) with a reference concentration of protein ARMET, wherein a concentration of protein ARMET above a reference concentration is indicative for an individual to be at risk for developing COPD.

### Prognosis

Prognostic indicators can be defined as clinical, pathological or biochemical features of COPD patients that predict with a certain likelihood the disease outcome. Their main use is to help to rationally plan patient management, i.e. to avoid undertreatment of aggressive disease and overtreatment of indolent disease, respectively.

As the level of protein ARMET alone significantly contributes to the differentiation of COPD patients from healthy controls or other diseases of the lung, e.g. asthma, bronchitis, pulmonary fibrosis and tuberculosis, preferably to differentiate COPD from asthma, it has to be expected that it will aid in assessing the prognosis of patients suffering from COPD. The concentration of protein ARMET will most likely be combined with results of lung function testing or spirometry.

### Differentiation of COPD from asthma

In a further embodiment the method according to the present invention is used to differentiate COPD from other types of lung diseases, preferably asthma.

It is also preferred to use the protein ARMET to differentiate COPD from other types of lung diseases, e.g. asthma, bronchitis, pulmonary fibrosis and tuberculosis, preferably to differentiate COPD from asthma. Surprisingly the inventors have found that the use of a marker combination of a COPD specific marker, preferably ARMET, and an inflammation marker selected from the group consisting of CRP, interleukin-6, serum amyloid A, S100 and E-selectin, can lead to a differentiation between COPD and other inflammatory diseases of the lung, e.g. asthma, acute or chronic inflammation of the lung, respectively. Experimental results for the protein ARMET and protein CRP are shown in the example section.

### Monitoring of disease progression

At present it is very difficult to predict with a reasonable likelihood whether a patient diagnosed with COPD has a more or less stable status or whether the disease will progress.

Progression of disease, i.e. of COPD, may be evaluated by in vitro monitoring of the concentration of protein ARMET in test samples, especially by taking one or more consecutive samples. As will be appreciated that an increase in the level of C-terminal proSP-B over time is indicative of disease progression.

### Monitor a patient's response to therapy

The method according to the present invention, when used in patient monitoring, may be used in the follow-up of patients and e.g. help to assess efficacy of a treatment of COPD.

Monitoring a patient's response to therapy can be practiced e.g. by establishing the pre- and post-therapeutic marker level for protein ARMET and by comparing the pre- and the post-therapeutic marker level.

A patient's response to a COPD treatment may be evaluated in vitro by monitoring the concentration of protein ARMET in test samples over time

The level of protein ARMET appears to be appropriate to monitor a patient's response to therapy. The present invention thus also relates to the use of protein ARMET in monitoring a patient's response to therapy, wherein a decreased level of protein ARMET is a positive indicator for an effective treatment of COPD.

### Marker combinations

The present invention therefore relates in an embodiment to the use of protein ARMET as one marker of a marker panel for the assessment of COPD. Such marker panel comprises protein ARMET and one or more additional marker for COPD. Certain combinations of markers will e.g. be advantageous in the screening for COPD.

As the skilled artisan will appreciate there are many ways to use the measurements of two or more markers in order to improve the diagnostic question under investigation.

Biochemical markers can either be determined individually or in an embodiment of the invention they can be determined simultaneously, e.g. using a chip or a bead based array technology. The concentrations of the biomarkers are then either interpreted independently, e.g., using an individual cut-off for each marker, or they are combined for interpretation.

As the skilled artisan will appreciate the step of correlating a marker level to a certain likelihood or risk can be performed and achieved in different ways. Preferably the determined concentration of protein ARMET and the one or more other marker(s) are mathematically combined and the combined value is correlated to the underlying diagnostic question. Marker values may be combined with the determination of ARMET by any appropriate state of the art mathematical method.

Preferably the mathematical algorithm applied in the combination of markers is a logistic function. The result of applying such mathematical algorithm or such logistical function preferably is a single value. Dependent on the underlying diagnostic question such value can easily be correlated to e.g., the risk of an individual for COPD or to other intended diagnostic uses helpful in the assessment of patients with COPD. In a preferred way such logistic function is obtained by a) classification of individuals into the groups, e.g., into normals, individuals at risk for COPD, patients with acute or chronic inflammation of the lung and so on, b) identification of markers which differ significantly between these groups by univariate analysis, c) logistic regression analysis to assess the independent discriminative values of markers useful in assessing these different groups and d) construction of the logistic function to combine the independent discriminative values. In this type of analysis the markers are no longer independent but represent a marker combination.

In an embodiment the logistic function used for combining the values for ARMET and the value of at least one further marker is obtained by a) classification of individuals into the groups of normals and individuals likely to have COPD, respectively, b) establishing the values for ARMET and the value of the at least one further marker c) performing logistic regression analysis and d) construction of the logistic function to combine the marker values for ARMET and the value of the at least one further marker.

A logistic function for correlating a marker combination to a disease preferably employs an algorithm developed and obtained by applying statistical methods. Appropriate statistical methods e.g. are Discriminant analysis (DA) (i.e., linear-, quadratic-, regularized-DA), Kernel Methods (i.e., SVM), Nonparametric Methods (i.e., k-Nearest-Neighbor Classifiers), PLS (Partial Least Squares), Tree-Based Methods (i.e., Logic Regression, CART, Random Forest Methods, Boosting/Bagging Methods), Generalized Linear Models (i.e., Logistic Regression), Principal Components based Methods (i.e., SIMCA), Generalized Additive Models, Fuzzy Logic based Methods, Neural Networks and Genetic Algorithms based Methods. The skilled artisan will have no problem in selecting an appropriate statistical method to evaluate a marker combination of the present invention and thereby to obtain an appropriate mathematical algorithm. In an embodiment the statistical method employed to obtain the mathematical algorithm used in the assessment of COPD is selected from DA (i.e., Linear-, Quadratic-, Regularized Discriminant Analysis), Kernel Methods (i.e., SVM), Nonparametric Methods (i.e., k-Nearest-Neighbor Classifiers), PLS (Partial Least Squares), Tree-Based Methods (i.e., Logic Regression, CART, Random Forest Methods, Boosting Methods), or Generalized Linear Models (i.e., Logistic Regression). Details relating to these statistical methods are found in the following references: Ruczinski, I., et al., J. of Computational and Graphical Statistics 12 (2003) 475-511; Friedman, J.H., J. of the American Statistical Association 84 (1989) 165-175; Hastie, T., et al., The Elements of Statistical Learning, Springer Verlag (2001); Breiman, L., et al., Classification and regression trees, Wadsworth International Group, California (1984); Breiman, L., Machine Learning 45 (2001) 5-32; Pepe, M.S., The Statistical Evaluation of Medical Tests for Classification and Prediction, Oxford Statistical Science Series, 28, Oxford University Press (2003); and Duda, R.O., et al., Pattern Classification, John Wiley & Sons, Inc., 2nd ed. (2001).

It is an embodiment of the invention to use an optimized multivariate cut-off for the underlying combination of biological markers and to discriminate state A from state B, e.g., normals and individuals at risk for COPD, COPD patients responsive to therapy and therapy failures, patients having an acute inflammation of the lung and COPD patients, COPD patients showing disease progression and COPD patients not showing disease progression, respectively.

The area under the receiver operator curve (=AUC) is an indicator of the performance or accuracy of a diagnostic procedure. Accuracy of a diagnostic method is best described by its receiver-operating characteristics (ROC) (see especially Zweig, M.H., and Campbell, G., Clin. Chem. 39 (1993) 561-577). The ROC graph is a plot of all of the sensitivity/specificity pairs resulting from continuously varying the decision thresh-hold over the entire range of data observed.

The clinical performance of a laboratory test depends on its diagnostic accuracy, or the ability to correctly classify subjects into clinically relevant subgroups. Diagnostic accuracy measures the test's ability to correctly distinguish two different conditions of the subjects investigated. Such conditions are for example, health and disease or disease progression versus no disease progression.

In each case, the ROC plot depicts the overlap between the two distributions by plotting the sensitivity versus 1 - specificity for the complete range of decision thresholds. On the y-axis is sensitivity, or the true-positive fraction [defined as (number of true-positive test results)/(number of true-positive + number of false-negative test results)]. This has also been referred to as positivity in the presence of a disease or condition. It is calculated solely from the affected subgroup. On the x-axis is the false-positive fraction, or 1 - specificity [defined as (number of false-positive results)/(number of true-negative + number of false-positive results)]. It is an index of specificity and is calculated entirely from the unaffected subgroup. Because the true- and false-positive fractions are calculated entirely separately, by using the test results from two different subgroups, the ROC plot is independent of the prevalence of disease in the sample. Each point on the ROC plot represents a sensitivity/1-specificity pair corresponding to a particular decision threshold. A test with perfect discrimination (no overlap in the two distributions of results) has an ROC plot that passes through the upper left corner, where the true-positive fraction is 1.0, or 100% (perfect sensitivity), and the false-positive fraction is 0 (perfect specificity). The theoretical plot for a test with no discrimination (identical distributions of results for the two groups) is a 45° diagonal line from the lower left corner to the upper right corner. Most plots fall in between these two extremes. (If the ROC plot falls completely below the 45° diagonal, this is easily remedied by reversing the criterion for "positivity" from "greater than" to "less than" or vice versa.) Qualitatively, the closer the plot is to the upper left corner, the higher the overall accuracy of the test.

One convenient goal to quantify the diagnostic accuracy of a laboratory test is to express its performance by a single number. The most common global measure is the area under the ROC plot (AUC). By convention, this area is always ≥ 0.5 (if it is not, one can reverse the decision rule to make it so). Values range between 1.0 (perfect separation of the test values of the two groups) and 0.5 (no apparent distributional difference between the two groups of test values). The area does not depend only on a particular portion of the plot such as the point closest to the diagonal or the sensitivity at 90% specificity, but on the entire plot. This is a quantitative, descriptive expression of how close the ROC plot is to the perfect one (area = 1.0).

The overall assay sensitivity will depend on the specificity required for practicing the method disclosed here. In certain preferred settings a specificity of 75% may be sufficient and statistical methods and resulting algorithms can be based on this specificity requirement. In one preferred embodiment the method used to assess individuals at risk for COPD is based on a specificity of 80%, of 85%, or also preferred of 90% or of 95%.

Certain combinations of markers will be advantageous in the screening for COPD.

In one embodiment the present invention is directed to an in vitro method for assessing COPD by biochemical markers, comprising determining in a sample the concentration of protein ARMET and of one or more other marker(s), mathematically combining the determined concentration of protein ARMET and the concentration of the one or more other marker, respectively, wherein a increased combined value is indicative for the presence of COPD.

In an embodiment the present invention is directed to an in vitro method for assessing COPD by biochemical markers, comprising determining in a serum, plasma, or whole blood sample the concentration of protein ARMET and of one or more other marker(s) and comparing the determined concentration of protein ARMET with a reference concentration of protein ARMET, wherein a concentration of protein ARMET above a reference concentration is indicative for the presence of COPD. It is preferred that the one or more other marker of said method is selected from the group consisting of ASC, NNMT, FEN1, APEX1 and Seprase. In a further preferred embodiment said marker panel comprises at least protein ARMET and protein NNMT.

In an embodiment the present invention relates to the use of marker ARMET as a marker molecule for the in vitro assessment of COPD in a human serum, plasma, or whole blood sample in combination with one or more marker molecule(s) indicative for COPD. The present invention therefore relates in an embodiment to the use of protein ARMET as one marker of a COPD marker panel, i.e. a marker panel comprising protein ARMET and one or more additional marker for COPD screening purposes.

For example the present invention also relates to the use of a marker panel comprising protein ARMET and ASC, or of a marker panel comprising protein ARMET and NNMT, or of a marker panel comprising protein ARMET and FEN1, or of a marker panel comprising protein ARMET and APEX1, or of a marker panel comprising protein ARMET and Seprase, or of a marker panel comprising protein ARMET and two or more markers selected from the group consisting of ASC, NNMT, FEN1, APEX1 and Seprase.

Markers for use in a combination with protein ARMET in the method according to the present invention are selected from the group consisting of ASC, NNMT, FEN1, APEX1 and Seprase. These markers may be used individually each or in any combination together with ARMET for assessing COPD.

In an embodiment the marker panel used in the in vitro method for assessing COPD by biochemical markers comprises the steps of determining in a serum, plasma, or whole blood sample the concentration of protein ARMET and of protein NNMT, wherein a concentration of protein ARMET above a reference concentration for protein ARMET is indicative for the presence of COPD. In a further embodiment the marker panel used in the in vitro method comprises the marker proteins ARMET, NNMT and Seprase. In a further embodiment the marker panel used in the in vitro method comprises the marker proteins ARMET, NNMT, Seprase and ASC.

### Marker of inflammation

Many serum markers for the diagnosis of an inflammation are presently known. The skilled artisan is familiar with the term "marker of inflammation". Said marker of inflammation is for example selected from the interleukin-6, C-reactive protein, serum amyloid A, sE-selectin and a S100 protein.

Interleukin-6 (IL-6) is a 21 kDa secreted protein that has numerous biological activities that can be divided into those involved in hematopoiesis and into those involved in the activation of the innate immune response. IL-6 is an acute-phase reactant and stimulates the synthesis of a variety of proteins, including adhesion molecules. Its major function is to mediate the acute phase production of hepatic proteins, and its synthesis is induced by the cytokines IL-1 and TNF-α. IL-6 is normally produced by macrophages and T lymphocytes. The normal serum concentration of IL-6 is < 5 pg/ml.

C-reactive protein (CRP) is a homopentameric Ca²⁺-binding acute phase protein with 21 kDa subunits that is involved in host defense. CRP synthesis is induced by IL-6, and indirectly by IL-1, since IL-1 can trigger the synthesis of IL-6 by Kupffer cells in the hepatic sinusoids. The normal plasma concentration of CRP is < 3 µg/ml (30 nM) in 90% of the healthy population, and < 10 µg/ml (100 nM) in 99% of healthy individuals. Plasma CRP concentrations can, e.g., be measured by an immunoassay. Plasma CRP concentrations can, e.g. be measured by homogeneous assay formats or ELISA.

Serum amyloid A (=SAA) is an acute phase protein of low molecular weight of 11.7 kDa. It is predominantly synthesized by the liver in response to IL-1, IL-6 or TNF-α stimulation and is involved in the regulation of the T-cell dependent immune response. Upon acute events the concentration of SAA increases up to 1000-fold reaching one milligram per milliliter. It is used to monitor inflammation in diseases as divers as cystic fibrosis, renal graft refection, trauma or infections. In rheumatoid arthritis is has in certain cases been used as a substitute for CRP, but, SAA is not yet as widely accepted.

S100-proteins form a constantly increasing family of Ca²⁺-binding proteins that today includes more than 20 members. The physiologically relevant structure of S100-proteins is a homodimer but some can also form heterodimers with each other, e.g., S100A8 and S100A9. The intracellular functions range from regulation of protein phosphorylation, of enzyme activities, or of the dynamics of the cytoskeleton to involvement in cell proliferation and differentiation. As some S100-proteins are also released from cells, extracellular functions have been described as well, e.g., neuronal survival, astrocyte proliferation, induction of apoptosis and regulation of inflammatory processes. S100A8, S100A9, the heterodimer S100A8/A9 and S100A12 have been found in inflammation with S100A8 responding to chronic inflammation, while S100A9, S100A8/A9 and S100A12 are increased in acute inflammation. S100A8, S100A9, S100A8/A9 and S100A12 have been linked to different diseases with inflammatory components including some cancers, renal allocraft rejection, colitis and most importantly to RA (Burmeister, G., and Gallacchi, G., Inflammopharmacology 3 (1995) 221-230; Foell, D., et al., Rheumathology 42 (2003) 1383-1389).

sE-selectin (soluble endothelial leukocyte adhesion molecule-1, ELAM-1) is a 115 kDa, type-I transmembrane glycoprotein expressed only on endothelial cells and only after activation by inflammatory cytokines (IL-1ß, TNF-α) or endotoxin. Cell-surface E-selectin is a mediator of the rolling attachment of leucocytes to the endothelium, an essential step in extravasion of leucocytes at the site of inflammation, thereby playing an important role in localized inflammatory response. Soluble E-selectin is found in the blood of healthy individuals, probably arising from proteolytic cleavage of the surface-expressed molecule. Elevated levels of sE-selectin in serum have been reported in a variety of pathological conditions (Gearing, A.J. and Hemingway, I., Ann. N.Y. Acad. Sci. 667 (1992) 324-331).

An array is a collection of addressable individual markers. Such markers can be spacially addressable, such as arrays contained within microtiter plates or printed on planar surfaces where each marker is present at distinct X and Y coordinates. Alternatively, markers can be addressable based on tags, beads, nanoparticles, or physical properties. A bio-chip array can be prepared according to the methods known to the ordinarily skilled artisan (see for example, US 5,807,522; Robinson, W.H., et al., Nat. Med. 8 (2002) 295-301; Robinson, W.H., et al., Arthritis Rheum. 46 (2002) 885-893). Array as used herein refers to any immunological assay with multiple addressable markers. A bio-chip array, also known to the skilled artisan as microarray, is a miniaturized form of an array.

The terms "chip", "bio-chip", "polymer-chip" or "protein-chip" are used interchangeably and refer to a collection of a large number of probes, markers or biochemical markers arranged on a shared substrate which could be a portion of a silicon wafer, a nylon strip, a plastic strip, or a glass slide.

An "array," "macroarray" or "microarray" is an intentionally created collection of substances, such as molecules, markers, openings, microcoils, detectors and/or sensors, attached to or fabricated on a substrate or solid surface, such as glass, plastic, silicon chip or other material forming an array. The arrays can be used to measure the levels of large numbers, e.g., tens, thousands or millions, of reactions or combinations simultaneously. An array may also contain a small number of substances, e.g., one, a few or a dozen. The substances in the array can be identical or different from each other. The array can assume a variety of formats, e.g., libraries of soluble molecules, libraries of immobilized molecules, libraries of immobilized antibodies, libraries of compounds tethered to resin beads, silica chips, or other solid supports. The array could either be a macroarray or a microarray, depending on the size of the pads on the array. A macroarray generally contains pad sizes of about 300 microns or larger and can be easily imaged by gel and blot scanners. A microarray would generally contain pad sizes of less than 300 microns.

A "solid support" is insoluble, functionalized, polymeric material to which library members or reagents may be attached or covalently bound (often via a linker) to be immobilized or allowing them to be readily separated (by filtration, centrifugation, washing etc.) from excess reagents, soluble reaction by- products, or solvents.

The following examples, sequence listing and figures are provided to aid the understanding of the present invention.

### Description of the Figures

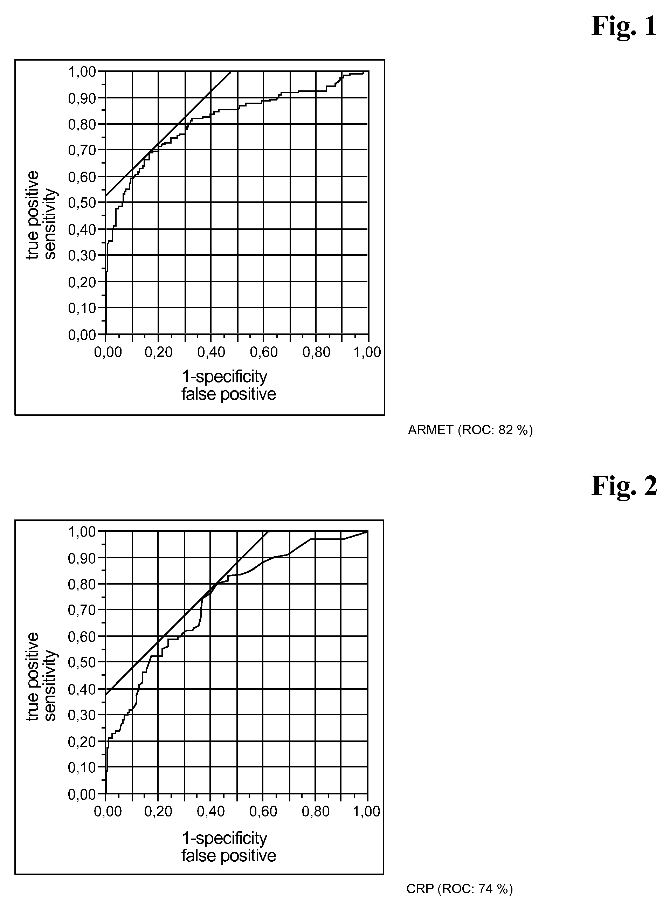

- **Figure 1**: Fig. 1 shows the plot of the receiver operator characteristics (ROC-plot) of protein ARMET in COPD samples with an AUC of 0.82 (ROC 82%) for the assessment of 123 samples obtained from patients with COPD as compared to 186 control samples obtained from healthy control patients. X-axis: 1-specificity (false positive); Y-axis: sensitivity (true positive).
- **Figure 2**: Fig. 2 shows the plot of the receiver operator characteristics (ROC-plot) of CRP in COPD samples with an AUC of 0.74 (ROC 74%) for the assessment of 123 samples obtained from patients with COPD as compared to 186 control samples obtained from healthy control patients. X-axis: 1-specificity (false positive); Y-axis: sensitivity (true positive).

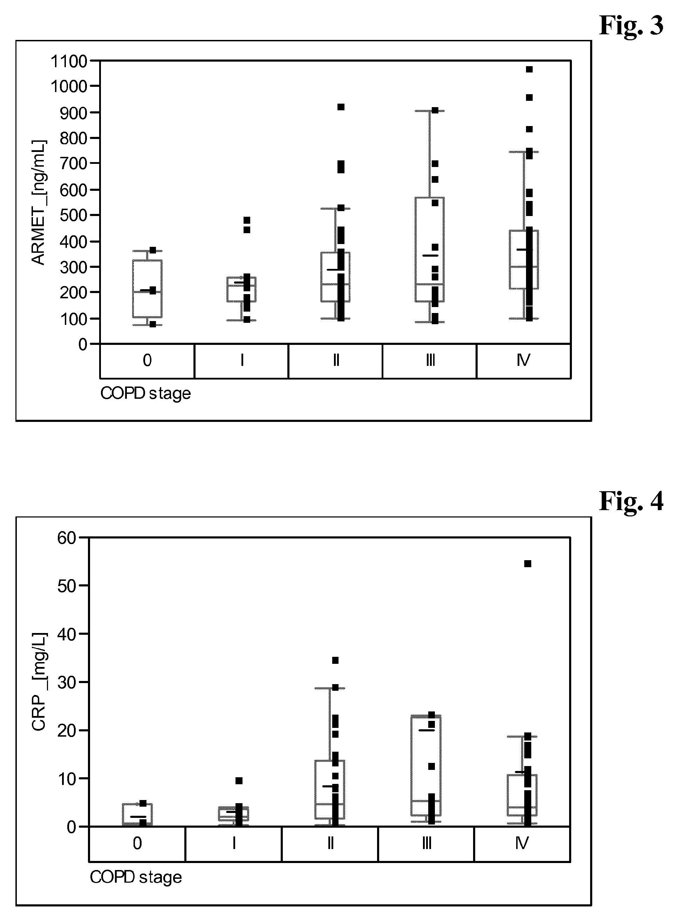

- **Figure 3**: Fig. 3 shows the box blot distribution of the determined ARMET serum concentration values according to the COPD stages 0 - IV of the 123 COPD samples (COPD stadium as described in Table 1).
- **Figure 4**: Fig. 4 shows the box plot distribution of the determined CRP serum concentration according to the COPD stages 0 - IV of the 123 COPD samples (COPD stadium as shown in Table 1).

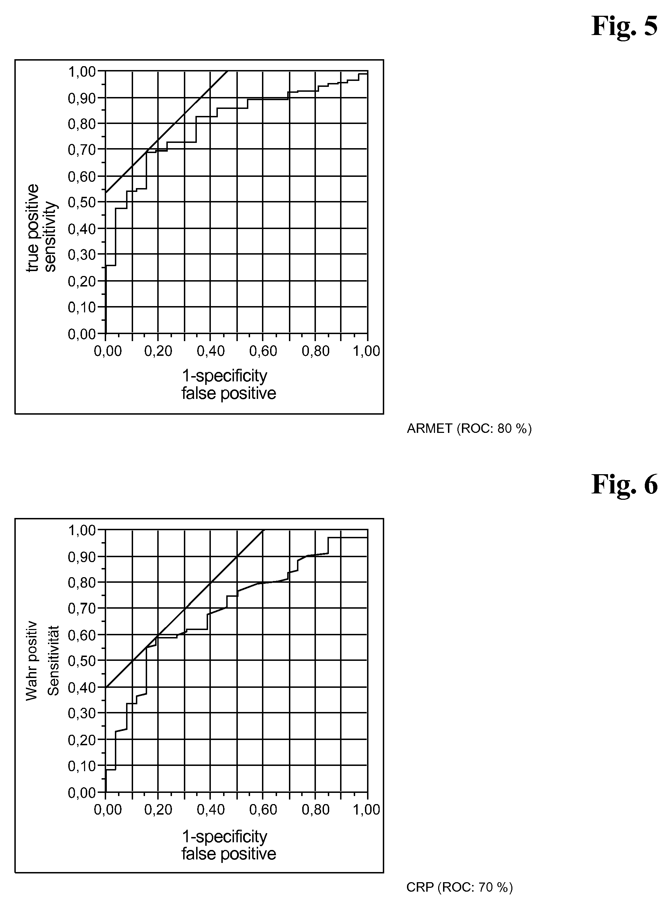

- **Figure 5**: Fig. 5 shows the plot of the receiver operator characteristics (ROC-plot) of protein ARMET in COPD samples with an AUC of 0.80 (ROC 80%) for the assessment of 123 samples obtained from patients with COPD as compared to 26 control samples obtained from patients with asthma. X-axis: 1-specificity (false positive); Y-axis: sensitivity (true positive).
- **Figure 6**: Fig. 6 shows the plot of the receiver operator characteristics (ROC-plot) of CRP in COPD samples with an AUC of 0.70 (ROC 70%) for the assessment of 123 samples obtained from patients with COPD as compared to 26 control samples obtained from patients with asthma. X-axis: 1-specificity (false positive); Y-axis: sensitivity (true positive).

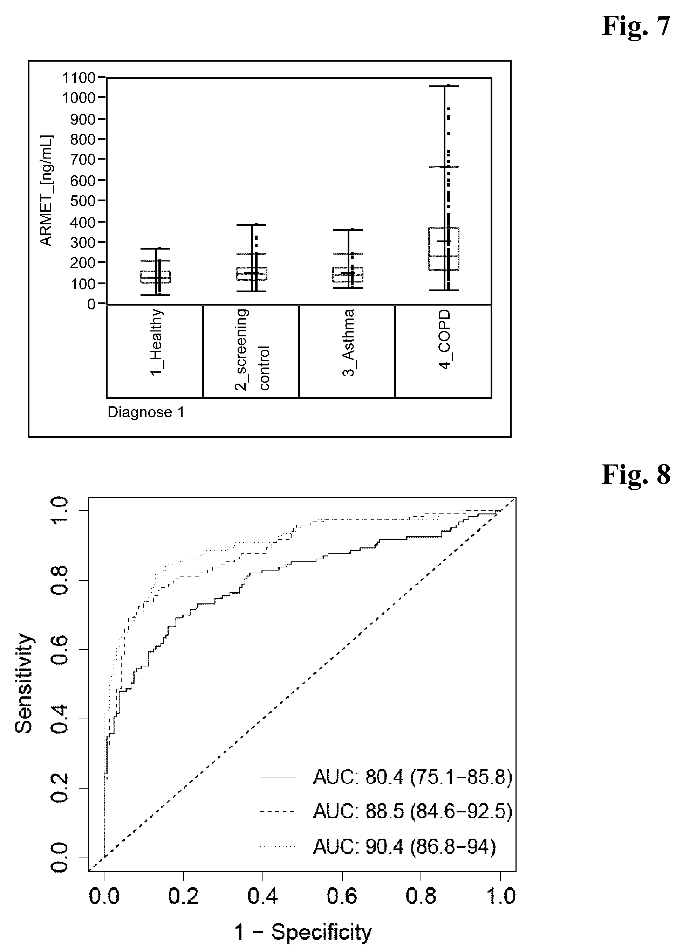

- **Figure 7**: Fig 7. shows a box plot distribution of the determined ARMET serum concentration [ng/ml] according to 123 COPD samples of stadium 0-IV (4_COPD), 50 healthy (1_Healthy), 135 screning controls (2_screening control) and 26 asthma patient samples (3_Asthma). The y-axis was adjusted for better 'visualization'.
- **Figure 8**: Fig. 8 shows the plot of the receiver operator characteristics (ROC-plot) of protein ARMET in COPD samples for ARMET (solid line), ARMET + NNMT (dashed line) and ARMET + NNMT + Seprase (dotted line) marker combinations for the assessment of 123 samples obtained from patients with COPD as compared to 161 control samples obtained from healthy control and asthma patients. X-axis: 1-specificity (false positive); Y-axis: sensitivity (true positive).

### Description of the Sequences

SEQ ID NO: 1 shows the amino acid sequence of the human protein ASC (SwissProt database accession number: Q9ULZ3).
SEQ ID NO: 2 shows the amino acid sequence of the human protein ARMET (SwissProt database accession number: P55145).
SEQ ID NO: 3 shows the amino acid sequence of the human protein NNMT (SwissProt database accession number: P40261).
SEQ ID NO: 4 shows the amino acid sequence of the human protein FEN1 (SwissProt database accession number: P39748).
SEQ ID NO: 5 shows the amino acid sequence of the human protein APEX1 (SwissProt database accession number: P27695).
SEQ ID NO: 6 shows the amino acid sequence of the human protein Seprase (SwissProt database accession number: Q12884).
SEQ ID NO: 7 shows the amino acid sequence of the human protein DPPIV (SwissProt database accession number: P27487).
SEQ ID NO: 8 Forward primer LC56for-*Eco*RI
SEQ ID NO: 9 Reverse primer LC56for-*Eco*RI
SEQ ID NO: 10 N-terminal peptide extension

### Example 1

### COPD study population

### Sources of serum samples:

In order to identify COPD-specific proteins as potential diagnostic markers for COPD, serum samples were derived from well-characterized patients with COPD (ATS classification system according table 1) in a national multi-center study. From each sample donor, spirometry was performed. Lung function, other diagnostic tests as well as reason for transferal, diagnosis and comorbidities were documented in a specific Case Report Form (CRF). The COPD samples have been evaluated in comparison with control samples obtained from control groups 1 - 4 as shown in table 2.

### Serum sample preparation:

Serum samples were drawn into a serum tube and allowed to clot for at least 60 minutes up to 120 minutes at room temperature. After centrifugation (10min, 2000g), the supernatant was divided into 1ml aliquots and frozen at -70°C. Before measurement, the samples were thawed, re-aliquoted into smaller volumes appropriate for prototype assays and reference assays and refrozen. Samples were thawed immediately before analysis. Therefore, each sample in the panel had only two freeze-thaw cycles before measurement.

### Example 2.1

### Generation of antibodies to protein ARMET

Polyclonal antibody to the marker protein ARMET is generated for further use of the antibody in the measurement of serum and plasma and blood levels of ARMET by immunodetection assays, e.g. Western Blotting and ELISA.

### Recombinant protein expression in E. coli:

In order to generate antibodies against ARMET, the recombinant antigen is produced in *E.coli:* Therefore, the ARMET coding region is PCR amplified from the full-length cDNA clone IRAUp969D0638D obtained from the German Resource Center for Genome Research (RZPD, Berlin, Germany) using the primers:

### Forward primer LC56for-EcoRI:

5' acgtacgtgaattcattaaagaggagaaattaactatATGAGAGGA TCGCATCACCATCACCATCACATTGAAGGCCGTAGGAGGATGTGGGCC ACGCAG (SEQ ID NO: 8 / *Eco*RI - site underlined, coding nucleotides in capital letters),

### Reverse primer LC56rev-BamHI:

5' acgtacgtggatcctcattaCAAATCGGTCCGTGCACTGG (SEQ ID NO: 9 / *Bam*HI - site underlined, coding nucleotides in capital letters).

The forward primer features (besides the *Eco*RI cloning and ribosomal binding sites) oligonucleotides coding for an N-terminal MRGSHHHHHHIEGR peptide extension (SEQ ID NO: 10) introduced in-frame to the ARMET protein. The *Eco*RI/*Bam*HI digested PCR fragment is ligated into the corresponding pQE-30 (Qiagen, Hilden, Germany) vector fragment which is subsequently transformed into *E.coli* XL1-blue competent cells. After sequence analysis, the plasmid is transformed into *E.coli* BL21 competent cells for expression under the IPTG - inducible T5 promoter of the pQE vector series following the manufacturer's instructions.

For purification of the MRGSHHHHHHIEGR-ARMET fusion protein, 1 1 of an over-night induced bacterial culture is pelleted by centrifugation and the cell pellet is lysed by resuspension in 100 mM sodium-phosphate buffer, pH 8.0, 7 M guanidium-hydrochloride, 5 mM imidazole, 20 mM thioglycerole. Insoluble material is pelleted by centrifugation and the supernatant is applied to Ni-nitrilotriacetic acid (Ni-NTA) metal-affinity chromatography: The column is washed with several bed volumes of lysis buffer followed by washes with a) 100 mM sodium-phosphate buffer, pH 8.0, 10 mM Tris-HCl, pH 8.0, 8 M urea, 20 mM thioglycerole; b) 100 mM sodium-phosphate buffer, pH 8.0, 0.5 % sodium-dodecylsulfate (SDS), 20 mM thioglycerole; and c) 100 mM sodium-phosphate buffer, pH 8.0, 0.1 % SDS, 20 mM thioglycerole. Finally, bound antigen is eluted using 100 mM sodium-phosphate buffer, pH 5.0, 0.1 % SDS, 20 mM thioglycerole, under acid conditions, and stored in the same buffer at 4°C.

### Generation of polyclonal antibodies:

### a) Immunization

For immunization, a fresh emulsion of the protein solution (100 µg/ml protein ARMET) and complete Freund's adjuvant at the ratio of 1:1 is prepared. Each rabbit is immunized with 1 ml of the emulsion at days 1,7, 14 and 30, 60 and 90. Blood is drawn and resulting anti-ARMET serum used for further experiments as described in examples 3 and 4.

### b) Purification of IgG (immunoglobulin G) from rabbit serum by sequential precipitation with caprylic acid and ammonium sulfate

One volume of rabbit serum is diluted with 4 volumes of acetate buffer (60 mM, pH 4.0). The pH is adjusted to 4.5 with 2 M Tris-base. Caprylic acid (25 µl/ml of diluted sample) is added drop-wise under vigorous stirring. After 30 min the sample is centrifuged (13 000 x g, 30 min, 4°C), the pellet discarded and the supernatant collected. The pH of the supernatant is adjusted to 7.5 by the addition of 2 M Tris-base and filtered (0.2 µm).

The immunoglobulin in the supernatant is precipitated under vigorous stirring by the drop-wise addition of a 4 M ammonium sulfate solution to a final concentration of 2 M. The precipitated immunoglobulins are collected by centrifugation (8000 x g, 15 min, 4°C).

The supernatant is discarded. The pellet is dissolved in 10 mM NaH₂PO₄/NaOH, pH 7.5, 30 mM NaCl and exhaustively dialyzed. The dialysate is centrifuged (13 000 x g, 15 min, 4°C) and filtered (0.2 µm).

### Biotinylation of polyclonal rabbit IgG:

Polyclonal rabbit IgG is brought to 10 mg/ml in 10 mM NaH₂PO₄/NaOH, pH 7.5, 30 mM NaCl. Per ml IgG solution 50 µl Biotin -N-hydroxysuccinimide (3.6 mg/ml in DMSO) are added. After 30 min at room temperature, the sample is chromatographed on Superdex 200 (10 mM NaH₂PO₄/NaOH, pH 7.5, 30 mM NaCl). The fraction containing biotinylated IgG are collected. Monoclonal antibodies have been biotinylated according to the same procedure.

### Digoxygenylation of polyclonal rabbit IgG:

Polyclonal rabbit IgG is brought to 10 mg/ml in 10 mM NaH₂PO₄/NaOH, 30 mM NaCl, pH 7.5. Per ml IgG solution 50 µl digoxigenin-3-O-methylcarbonyl-ε-aminocaproic acid-N-hydroxysuccinimide ester (Roche Diagnostics, Mannheim, Germany, Cat. No. 1 333 054) (3.8 mg/ml in DMSO) are added. After 30 min at room temperature, the sample is chromatographed on Superdex® 200 (10 mM NaH₂PO₄/NaOH, pH 7.5, 30 mM NaCl). The fractions containing digoxigenylated IgG are collected. Monoclonal antibodies have been labeled with digoxigenin according to the same procedure.

### Example 2.2

### CRP

The marker protein CRP is measured using a homogenous assay (Hitachi) distributed by Roche Diagnostics, Mannheim (FRG).

### Example 3

### ELISA for the measurement of ARMET in human serum or plasma samples

For detection of ARMET in human serum or plasma samples, a sandwich ELISA was developed. For capture and detection of the antigen, aliquots of the antibody against ARMET were conjugated with biotin and digoxygenin, respectively.

Samples (20µl) were mixed in separate wells of a streptavidin-coated microtiter plate with 100 µl of antibody reagent containing 0.12 µg/ml of each, biotin labeled and digoxigenin labeled antibodies in incubation buffer (40 mM phosphate, 200 mM sodium tartrate, 10 mM EDTA, 0.05% phenol, 0.1% polyethylene glycol 40000, 0.1% Tween 20, 0.2% BSA, 0.1% bovine IgG, 0.02% 5-Bromo-5-Nitro-1,3-Dioxane adjusted to pH 7.4, supplemented with 200 µg/ml polymeric monoclonal mouse IgG Fab-fragments for elimination of human anti-rat antibody response (HARA); Roche Diagnostics GmbH, Mannheim, Germany, Catalog # 11096478-001).

After incubation for one hour plates were washed three times with washing buffer (10 mM Tris, 150 mM NaCl, 0.05% Tween 20).

In a next step, wells were incubated with 30 mU/ml anti-digoxigenin-HRP conjugate (Roche Diagnostics GmbH, Mannheim, Germany, Catalog # 1633716) in Universal Conjugate Buffer (Roche Diagnostics GmbH, Mannheim, Germany, Catalog # 11684825) for 60 min and washed as before.

Wells were then incubated for 30 min. with 100 µl of TMB substrate solution (Roche Diagnostics GmbH, Mannheim, Germany, Catalog # 12034425). Adding of 2N sulfuric acid (50 µl) stopped the color development and switched the blue color into yellow. OD was measured at 450 nm with an ELISA reader.

All incubations were at room temperature. Samples of human serum or plasma were pre-diluted with incubation buffer ad 5 %. For calibration, a human serum was used as a standard. It was diluted with incubation buffer ad 2/4/8/16/32% to make calibrators with arbitrarily given values of 2/4/8/16/32 Units/ml, respectively.

The equation of the calibration curve was calculated by non-linear least-squares curve-fitting (Wiemer-Rodbard) and used for converting the absorbance reading of a well into the corresponding concentration value. The result was multiplied by the pre-dilution factor to get the concentration of the respective sample itself.

### Example 4

### ARMET as a serum marker for COPD

Serum samples derived from 123 well-characterized COPD patients of the ATS COPD stage 0 - IV classification shown in table 1 are used. The study population is shown in Table 2.

**Table 2: Study population**

| Sample type | Number of samples |
|---|---|
| COPD Stage 0 - IV (according to ATS classification shown in table 1) | 123 |
| Control 1: healthy nonsmokers (normal lung function) | 50 |
| Control 2: healthy smokers & former smokers (normal lung function) | 88 |
| Control 3: healthy individuals with occupational risk (asbestos, silica, dust,...) | 48 |
| Control 4: asthma patients | 26 |

The serum concentration of protein ARMET in the COPD samples is evaluated in comparison to control samples (Control 1, 2 and 3) obtained from obviously healthy individuals (= control cohort), and asthma patients (Control 4), with an AUC of 0.82 (Table 3). A receiver operator characteristic curve (ROC) of the results represented in Table 3 of marker ARMET is shown in Fig. 1. Data determined for the inflammation marker CRP are shown in Fig. 2. The AUC of marker ARMET is higher than the AUC of CRP.

**Table 3: ROC analysis of the marker protein in comparison to CRP**

| Marker | ARMET | CRP |
|---|---|---|
| ROC | 82 % | 74 % |

The cut-off value was determined in the control collective by calculation of the 95% quantile resulting in a 95% specificity. The diagnostic potential of the biomarker was evaluated either by calculating the receiver operator characteristic curves (ROC) (Table 3) or the clinical sensitivity at the preset specificity of 95% (Table 4). The sensitivity for a cut-off vs healthy individuals (Control 1) for COPD of marker ARMET is 63%. With a cut-off value that yields 95% specificity on the respective control cohort (Control 1, 2 and 3: namely healthy nonsmokers, smokers, former smokers and individuals with occupational risk to develop COPD), the sensitivity of marker ARMET for a cut-off for general screening for COPD is 48%.

**Table 4: Sensitivity and specificity of the marker protein in comparison to CRP**

| Marker | ARMET | CRP |
|---|---|---|
| specificity | 95 % | 95 % |
| sensitivity (cut-off control 1) | 63 % | 31 % |
| sensitivity (cut-off control 1, 2 and 3) | 48 % | 24 % |

When applying a cut-off (95 % specificity) based on control 1 (healthy control according to table 2) or based on control 1, 2 and 3 (screening controls according to table 2), the sensitivity of marker ARMET is higher than the sensitivity of CRP (Table 4). This is also reflected by ROC analysis, wherein marker ARMET exhibit a greater AUC than the marker CRP (Table 3).

The data determined for protein ARMET in COPD samples according ATS COPD stages 0 - IV have been used to calculate the box-plot shown in Fig. 3, representing the correlation of the serum concentration of protein ARMET with the ATS COPD stages 0 - IV. The data determined for the inflammation marker CRP within each sample classified according to the ATS COPD stages 0 - IV have been used to calculate the box-plot shown in Fig. 4, representing the correlation of the serum concentration of CRP with the COPD stadium.

The marker ARMET shows a weak correlation with the ATS stages 0 - IV. Therefore the ARMET serum concentration could be used for COPD diagnosis, but not for COPD staging.

### Example 5

### ARMET as a serum marker to differentiate human COPD vs Asthma

Samples derived from 123 well-characterized COPD patients according to ATS COPD stage 0 - IV classification shown in table 1 as well as samples derived from 26 asthma patients (Control 4 as shown in Table 2) were analysed using the marker ARMET. With a cut-off value that yields 95% specificity vs the asthma control cohort, the sensitivity for COPD is 49% (Table 5).

The sensitivity to differentiate COPD from asthma of marker ARMET is higher than the sensitivity of the inflammation marker CRP.

**Table 5: Differentiation of COPD vs asthma by usage of marker protein**

| Marker | ARMET | CRP |
|---|---|---|
| specificity (vs. asthma) | 95 % | 95 % |
| sensitivity (for COPD) | 49 % | 25 % |
| ROC | 80 % | 70 % |

A graphical representation of the results of marker ARMET is shown in Fig. 5 as a receiver operator characteristic curves (ROC). The results for the inflammation marker CRP is shown in Fig. 6 as a receiver operator characteristic curves (ROC).

The data determined for protein ARMET in COPD samples have been used to calculate the box-plot shown in Fig. 7 based on the data shown in Table 6, representing the correlation of the serum concentration of protein ARMET with the ATS COPD stages 0 - IV (n=123, as shown in Table 2) vs samples from healthy subjects (n=50), samples from screening control (n=135) and asthma patients (n=26). While mean values of controls (healthy, screening control and asthma) range between 137 and 158 ng/ml, ARMET concentrations of COPD patients are significantly higher with a mean value of 310 ng/ml. Results are represented in Table 6.

**Table 6: Variability of ARMET**

| ARMET | N | minimum [ng/mL] | maximum [ng/mL] | mean value [ng/mL] | std. div. | std. error mean value | 95% KI lower | 95% KI upper |
|---|---|---|---|---|---|---|---|---|
| 1_Healthy | 50 | 48.862 | 272.861 | 137.0542 | 41.91579 | 5.927788 | 125.1419 | 226.7957 |
| 2_Screening control | 135 | 65.296 | 388.932 | 158.0733 | 54.72844 | 4.710274 | 148.7572 | 148.9666 |
| 3_Asthma | 26 | 83.866 | 368.592 | 158.3229 | 60.9101 | 11.94545 | 133.7208 | 167.3894 |
| 4_COPD | 123 | 72.812 | 1061.44 | 309.7278 | 199.509 | 17.98912 | 274.1165 | 182.925 |

### Example 6

### Marker combinations / statistical analysis and results

Penalized Logistic Regression (PLR) was used as a mathematical model for marker combinations as implemented in the R-toolbox "glmnet" (http://cran.r-project.org/). To search for an additional marker, the initial marker entered in an unpenalized way the model, whereas all other markers were subject to penalization.

The algorithm optimisation (namely the selection of the penalization type and its penalization parameter) was carried out by an internal repeated 10-fold cross-validation, whereas the derivation of the performance parameters (sensitivity and specificity) was based on an outer repeated 10-fold cross-validation.

The original dataset was split into 10 parts, afterwards 9 of these parts formed the training-set and the 10th part the test set. The training set was then also split into 10 parts, were 9 of these parts formed the sub-training set and the 10th part the sub-testset. With these sub-datasets the penalization parameter was optimized based on the number of additional markers. With this optimized value the PLR was applied on the whole training set to generate a diagnostic rule. A threshold on the estimated posterior case-probabilities was determined on the controls as well as on the cases of the training set to achieve an apparent specificity and sensitivity of 90% for the multivariate diagnostic rule. This rule was then applied to the test set to estimate sensitivity and specificity at the given threshold. The external 10-fold cross-validation was repeated 50 times, the internal cross-validation 25 times.

A close analysis of the individual runs from cross validation revealed that the best additional marker for ARMET is NNMT, as it was selected as best additional marker in all runs. The best model with two additional markers is ARMET plus NNMT and Seprase. The best model with three additional markers is ARMET plus NNMT, Seprase and ASC.

Samples derived from 123 well-characterized COPD patients according to ATS COPD stage 0 - IV classification, as shown in table 2, as well as a control cohort consisting of 161 samples derived from healthy (n=136) and asthma patients (n=25) were analysed.

In Table 7 the classification performance for these combinations on training and testset are given, based on a specificity of 90%.

The results in Table 7 clearly show, that by combination of one additional marker the sensitivity can be significantly improved compared to ARMET as single marker without any loss of specificity.

**Table 7: Marker combinations on a specificity of 90%**

| Combination | Train. Sens. | Train Spec. | Test. Sens. | Test Spec. |
|---|---|---|---|---|
| | [log] | [log] | [log] | [log] |
| ARMET+NNMT | 0.73 | 0.9 | 0.73 | 0.89 |
| | (0.7-0.77) | (0.89-0.9) | (0.72-0.75) | (0.89-0.91) |
| ARMET+NNMT+Seprase | 0.74 | 0.9 | 0.73 | 0.89 |
| | (0.69-0.79) | (0.89-0.9) | (0.7-0.75) | (0.85-0.91) |
| ARMET+NNMT+Seprase+ASC | 0.82 | 0.9 | 0.8 | 0.88 |
| | (0.73-0.86) | (0.89-0.9) | (0.75-0.83) | (0.86-0.9) |

In Table 8 the classification performance for these combinations on training and testset are given, based on a sensitivity of 90%. The results in Table 8 clearly show, that by combination of one additional marker the specificity can be significantly improved compared to ARMET as single marker without any loss of sensitivity.

**Table 8: Marker combinations on a sensitivity of 90%**

| Combination | Train. Sens. [log] | Train Spec. [log] | Test. Sens. [log] | Test Spec. [log] |
|---|---|---|---|---|
| ARMET+NNMT | 0.9 (0.89-0.9) | 0.58 (0.55-0.64) | 0.89 (0.87-0.9) | 0.58 (0.58-0.6) |
| ARMET+NNMT+Seprase | 0.9 (0.89-0.9) | 0.68 (0.6-0.76) | 0.89 (0.86-0.9) | 0.68 (0.65-0.72) |
| ARMET+NNMT+Seprase+ASC | 0.9 (0.89-0.9) | 0.76 (0.66-0.82) | 0.87 (0.84-0.89) | 0.75 (0.72-0.78) |

With a cut-off value that yields 90% specificity vs control cohort, the sensitivity for a cut-off for general screening with ARMET is 80.4 %, with ARMET + NNMT is 88.5%, with ARMET + NNMT + Seprase is 90.4% and with ARMET + NNMT + Seprase + ASC is 93.3% (4 marker combination not shown in Fig. 8). A graphical representation of the results of marker ARMET and marker combinations for up to 3 markers is shown in Fig. 8 as a receiver operator characteristic curves (ROC).

## Claims

1. An in vitro method for assessing chronic obstructive pulmonary disease (COPD) in a human subject, comprising
a) determining the concentration of protein ARMET in a serum, plasma, or whole blood sample, and
b) comparing the concentration of protein ARMET determined in step (a) with a reference concentration of protein ARMET, wherein a concentration of protein ARMET above a reference concentration is indicative for COPD.

2. The method according to claim 1, wherein the protein ARMET is measured in an immunoassay procedure.

3. The method according to claim 2, wherein the immunoassay procedure is an enzyme-linked immunoassay (ELISA).

4. The method according to claims 2 and 3, wherein ARMET is measured in a sandwich assay format.

5. The method according to claims 2 and 3, wherein ARMET is measured in a competitive assay format.

6. Use of protein ARMET in the in vitro assessment of COPD in a human serum, plasma, or whole blood sample, wherein a concentration of protein ARMET above a reference concentration for protein ARMET is indicative for COPD.

7. Use of a marker panel comprising protein ARMET and protein NNMT in the in vitro assessment of COPD in a human serum, plasma, or whole blood sample, wherein a concentration of protein ARMET above a reference concentration for protein ARMET is indicative for COPD.

8. Use of the marker panel according to claim 7 comprising proteins ARMET, NNMT and Seprase.

9. Use of the marker panel according to claim 7 comprising proteins ARMET, NNMT, Seprase and ASC.

10. Use of a method according to any one of the claims 1 to 5 to differentiate COPD from asthma.

## Patentansprüche

1. In-vitro-Verfahren zur Beurteilung von chronisch obstruktiver Lungenerkrankung (Chronic Obstructive Pulmonary Disease, COPD) bei einem menschlichen Individuum, umfassend
a) Bestimmen der Konzentration von Protein ARMET in einer Serum-, Plasma- oder Vollblutprobe und
b) Vergleichen der in Schritt (a) bestimmten Konzentration von Protein ARMET mit einer Referenzkonzentration von Protein ARMET, wobei eine Konzentration von Protein ARMET oberhalb einer Referenzkonzentration COPD anzeigt.

2. Verfahren nach Anspruch 1, wobei das Protein ARMET in einem Immuntestverfahren gemessen wird.

3. Verfahren nach Anspruch 2, wobei es sich bei dem Immuntestverfahren um einen ELISA-Test (Enzyme-linked Immunoassay) handelt.

4. Verfahren nach Anspruch 2 und 3, wobei ARMET in einem Sandwich-Testformat gemessen wird.

5. Verfahren nach Anspruch 2 und 3, wobei ARMET in einem kompetitiven Testformat gemessen wird.

6. Verwendung von Protein ARMET bei der In-vitro-Beurteilung von COPD in einer menschlichen Serum-, Plasma- oder Vollblutprobe, wobei eine Konzentration von Protein ARMET oberhalb einer Referenzkonzentration für Protein ARMET COPD anzeigt.

7. Verwendung einer Marker-Palette, umfassend Protein ARMET und Protein NNMT, bei der In-vitro-Beurteilung von COPD in einer menschlichen Serum-, Plasma- oder Vollblutprobe, wobei eine Konzentration von Protein ARMET oberhalb einer Referenzkonzentration für Protein ARMET COPD anzeigt.

8. Verwendung der Marker-Palette nach Anspruch 7, umfassend die Proteine ARMET, NNMT und Seprase.

9. Verwendung der Marker-Palette nach Anspruch 7, umfassend die Proteine ARMET, NNMT, Seprase und ASC.

10. Verwendung eines Verfahrens nach einem der Ansprüche 1 bis 5 zur Unterscheidung von COPD von Asthma.

## Revendications

1. Procédé *in vitro* d'évaluation de la maladie pulmonaire obstructive chronique (MPOC) chez un sujet humain, comprenant
a) la détermination de la concentration de protéine ARMET dans un prélèvement de sérum, de plasma, ou de sang total humain, et
b) la comparaison de la concentration de la protéine ARMET déterminée dans l'étape (a) à une concentration de référence de protéine ARMET, une concentration de la protéine ARMET située au-dessus d'une concentration de référence étant indicative de la MPOC.

2. Procédé selon la revendication 1, la protéine ARMET étant mesurée dans une procédure d'immunoessai.

3. Procédé selon la revendication 2, la procédure d'immunoessai étant un dosage par immunosorbent lié à une enzyme (ELISA).

4. Procédé selon les revendications 2 et 3, l'ARMET étant mesurée dans un format de dosage en sandwich.

5. Procédé selon les revendications 2 et 3, l'ARMET étant mesurée dans un format de dosage compétitif.

6. Utilisation de protéine ARMET dans l'évaluation *in vitro* de la MPOC dans un prélèvement de sérum, de plasma, ou de sang total humain, une concentration de protéine ARMET située au-dessus d'une concentration de référence pour la protéine ARMET étant indicative de la MPOC.

7. Utilisation d'un panel de marqueurs comprenant la protéine ARMET et la protéine NNMT dans l'évaluation *in vitro* de la MPOC dans un prélèvement de sérum, de plasma, ou de sang total humain, une concentration de la protéine ARMET située au-dessus d'une concentration de référence pour la protéine ARMET étant indicative de la MPOC.

8. Utilisation du panel de marqueurs selon la revendication 7 comprenant les protéines ARMET, NNMT et séprase.

9. Utilisation du panel de marqueurs selon la revendication 7 comprenant les protéines ARMET, NNMT, séprase et ASC.

10. Utilisation d'un procédé selon l'une quelconque des revendications 1 à 5 pour différencier la MPOC de l'asthme.


### SEQUENCE LISTING

<110> Roche Diagnostics GmbH
   F. Hoffmann-La Roche AG
<120> ARMET as marker for chronic obstructive pulmonary disease (COPD)
<130> 27347 WO-TH
<150> EP11157918.1
   <151> 2011-03-11
<160> 10
<170> PatentIn version 3.5
<210> 1
   <211> 195
   <212> PRT
   <213> Homo sapiens
<400> 1
   Met Gly Arg Ala Arg Asp Ala Ile Leu Asp Ala Leu Glu Asn Leu Thr
   1               5                   10                  15
   Ala Glu Glu Leu Lys Lys Phe Lys Leu Lys Leu Leu Ser Val Pro Leu
               20                  25                  30
   Arg Glu Gly Tyr Gly Arg Ile Pro Arg Gly Ala Leu Leu Ser Met Asp
           35                  40                  45
   Ala Leu Asp Leu Thr Asp Lys Leu Val Ser Phe Tyr Leu Glu Thr Tyr
       50                  55                  60
   Gly Ala Glu Leu Thr Ala Asn Val Leu Arg Asp Met Gly Leu Gln Glu
   65                  70                  75                  80
   Met Ala Gly Gln Leu Gln Ala Ala Thr His Gln Gly Ser Gly Ala Ala
                   85                  90                  95
   Pro Ala Gly Ile Gln Ala Pro Pro Gln Ser Ala Ala Lys Pro Gly Leu
               100                 105                 110
   His Phe Ile Asp Gln His Arg Ala Ala Leu Ile Ala Arg Val Thr Asn
           115                 120                 125
   Val Glu Trp Leu Leu Asp Ala Leu Tyr Gly Lys Val Leu Thr Asp Glu
       130                 135                 140
   Gln Tyr Gln Ala Val Arg Ala Glu Pro Thr Asn Pro Ser Lys Met Arg
   145                 150                 155                 160
   Lys Leu Phe Ser Phe Thr Pro Ala Trp Asn Trp Thr Cys Lys Asp Leu
                   165                 170                 175
   Leu Leu Gln Ala Leu Arg Glu Ser Gln Ser Tyr Leu Val Glu Asp Leu
               180                 185                 190
   Glu Arg Ser
           195
<210> 2
   <211> 179
   <212> PRT
   <213> Homo sapiens
<400> 2
<210> 3
   <211> 264
   <212> PRT
   <213> Homo sapiens
<400> 3
   Met Glu Ser Gly Phe Thr Ser Lys Asp Thr Tyr Leu Ser His Phe Asn
   1               5                   10                  15
   Pro Arg Asp Tyr Leu Glu Lys Tyr Tyr Lys Phe Gly Ser Arg His Ser
               20                  25                  30
   Ala Glu Ser Gln Ile Leu Lys His Leu Leu Lys Asn Leu Phe Lys Ile
           35                  40                  45
   Phe Cys Leu Asp Gly Val Lys Gly Asp Leu Leu Ile Asp Ile Gly Ser
       50                  55                  60
   Gly Pro Thr Ile Tyr Gln Leu Leu Ser Ala Cys Glu Ser Phe Lys Glu
   65                  70                  75                  80
   Ile Val Val Thr Asp Tyr Ser Asp Gln Asn Leu Gln Glu Leu Glu Lys
                   85                  90                  95
   Trp Leu Lys Lys Glu Pro Glu Ala Phe Asp Trp Ser Pro Val Val Thr
               100                 105                 110
   Tyr Val Cys Asp Leu Glu Gly Asn Arg Val Lys Gly Pro Glu Lys Glu
           115                 120                 125
   Glu Lys Leu Arg Gln Ala Val Lys Gln Val Leu Lys Cys Asp Val Thr
       130                 135                 140
   Gln Ser Gln Pro Leu Gly Ala Val Pro Leu Pro Pro Ala Asp Cys Val
   145                 150                 155                 160
   Leu Ser Thr Leu Cys Leu Asp Ala Ala Cys Pro Asp Leu Pro Thr Tyr
                   165                 170                 175
   Cys Arg Ala Leu Arg Asn Leu Gly Ser Leu Leu Lys Pro Gly Gly Phe
               180                 185                 190
   Leu Val Ile Met Asp Ala Leu Lys Ser Ser Tyr Tyr Met Ile Gly Glu
           195                 200                 205
   Gln Lys Phe Ser Ser Leu Pro Leu Gly Arg Glu Ala Val Glu Ala Ala
       210                 215                 220
   Val Lys Glu Ala Gly Tyr Thr Ile Glu Trp Phe Glu Val Ile Ser Gln
   225                 230                 235                 240
   Ser Tyr Ser Ser Thr Met Ala Asn Asn Glu Gly Leu Phe Ser Leu Val
                   245                 250                 255
   Ala Arg Lys Leu Ser Arg Pro Leu
               260
<210> 4
   <211> 380
   <212> PRT
   <213> Homo sapiens
<400> 4
   Met Gly Ile Gln Gly Leu Ala Lys Leu Ile Ala Asp Val Ala Pro Ser
   1               5                   10                  15
   Ala Ile Arg Glu Asn Asp Ile Lys Ser Tyr Phe Gly Arg Lys Val Ala
               20                  25                  30
   Ile Asp Ala Ser Met Ser Ile Tyr Gln Phe Leu Ile Ala Val Arg Gln
           35                  40                  45
   Gly Gly Asp Val Leu Gln Asn Glu Glu Gly Glu Thr Thr Ser His Leu
       50                  55                  60
   Met Gly Met Phe Tyr Arg Thr Ile Arg Met Met Glu Asn Gly Ile Lys
   65                  70                  75                  80
   Pro Val Tyr Val Phe Asp Gly Lys Pro Pro Gln Leu Lys Ser Gly Glu
                   85                  90                  95
   Leu Ala Lys Arg Ser Glu Arg Arg Ala Glu Ala Glu Lys Gln Leu Gln
               100                 105                 110
   Gln Ala Gln Ala Ala Gly Ala Glu Gln Glu Val Glu Lys Phe Thr Lys
           115                 120                 125
   Arg Leu Val Lys Val Thr Lys Gln His Asn Asp Glu Cys Lys His Leu
       130                 135                 140
   Leu Ser Leu Met Gly Ile Pro Tyr Leu Asp Ala Pro Ser Glu Ala Glu
   145                 150                 155                 160
   Ala Ser Cys Ala Ala Leu Val Lys Ala Gly Lys Val Tyr Ala Ala Ala
                   165                 170                 175
   Thr Glu Asp Met Asp Cys Leu Thr Phe Gly Ser Pro Val Leu Met Arg
               180                 185                 190
   His Leu Thr Ala Ser Glu Ala Lys Lys Leu Pro Ile Gln Glu Phe His
           195                 200                 205
   Leu Ser Arg Ile Leu Gln Glu Leu Gly Leu Asn Gln Glu Gln Phe Val
       210                 215                 220
   Asp Leu Cys Ile Leu Leu Gly Ser Asp Tyr Cys Glu Ser Ile Arg Gly
   225                 230                 235                 240
   Ile Gly Pro Lys Arg Ala Val Asp Leu Ile Gln Lys His Lys Ser Ile
                   245                 250                 255
   Glu Glu Ile Val Arg Arg Leu Asp Pro Asn Lys Tyr Pro Val Pro Glu
               260                 265                 270
   Asn Trp Leu His Lys Glu Ala His Gln Leu Phe Leu Glu Pro Glu Val
           275                 280                 285
   Leu Asp Pro Glu Ser Val Glu Leu Lys Trp Ser Glu Pro Asn Glu Glu
       290                 295                 300
   Glu Leu Ile Lys Phe Met Cys Gly Glu Lys Gln Phe Ser Glu Glu Arg
   305                 310                 315                 320
   Ile Arg Ser Gly Val Lys Arg Leu Ser Lys Ser Arg Gln Gly Ser Thr
                   325                 330                 335
   Gln Gly Arg Leu Asp Asp Phe Phe Lys Val Thr Gly Ser Leu Ser Ser
               340                 345                 350
   Ala Lys Arg Lys Glu Pro Glu Pro Lys Gly Ser Thr Lys Lys Lys Ala
           355                 360                 365
   Lys Thr Gly Ala Ala Gly Lys Phe Lys Arg Gly Lys
       370                 375                 380
<210> 5
   <211> 318
   <212> PRT
   <213> Homo sapiens
<400> 5
   Met Pro Lys Arg Gly Lys Lys Gly Ala Val Ala Glu Asp Gly Asp Glu
   1               5                   10                  15
   Leu Arg Thr Glu Pro Glu Ala Lys Lys Ser Lys Thr Ala Ala Lys Lys
               20                  25                  30
   Asn Asp Lys Glu Ala Ala Gly Glu Gly Pro Ala Leu Tyr Glu Asp Pro
           35                  40                  45
   Pro Asp Gln Lys Thr Ser Pro Ser Gly Lys Pro Ala Thr Leu Lys Ile
       50                  55                  60
   Cys Ser Trp Asn Val Asp Gly Leu Arg Ala Trp Ile Lys Lys Lys Gly
   65                  70                  75                  80
   Leu Asp Trp Val Lys Glu Glu Ala Pro Asp Ile Leu Cys Leu Gln Glu
                   85                  90                  95
   Thr Lys Cys Ser Glu Asn Lys Leu Pro Ala Glu Leu Gln Glu Leu Pro
               100                 105                 110
   Gly Leu Ser His Gln Tyr Trp Ser Ala Pro Ser Asp Lys Glu Gly Tyr
           115                 120                 125
   Ser Gly Val Gly Leu Leu Ser Arg Gln Cys Pro Leu Lys Val Ser Tyr
       130                 135                 140
   Gly Ile Gly Asp Glu Glu His Asp Gln Glu Gly Arg Val Ile Val Ala
   145                 150                 155                 160
   Glu Phe Asp Ser Phe Val Leu Val Thr Ala Tyr Val Pro Asn Ala Gly
                   165                 170                 175
   Arg Gly Leu Val Arg Leu Glu Tyr Arg Gln Arg Trp Asp Glu Ala Phe
               180                 185                 190
   Arg Lys Phe Leu Lys Gly Leu Ala Ser Arg Lys Pro Leu Val Leu Cys
           195                 200                 205
   Gly Asp Leu Asn Val Ala His Glu Glu Ile Asp Leu Arg Asn Pro Lys
       210                 215                 220
   Gly Asn Lys Lys Asn Ala Gly Phe Thr Pro Gln Glu Arg Gln Gly Phe
   225                 230                 235                 240
   Gly Glu Leu Leu Gln Ala Val Pro Leu Ala Asp Ser Phe Arg His Leu
                   245                 250                 255
   Tyr Pro Asn Thr Pro Tyr Ala Tyr Thr Phe Trp Thr Tyr Met Met Asn
   260 265 270
   Ala Arg Ser Lys Asn Val Gly Trp Arg Leu Asp Tyr Phe Leu Leu Ser
   275 280 285
   His Ser Leu Leu Pro Ala Leu Cys Asp Ser Lys Ile Arg Ser Lys Ala
   290 295 300
   Leu Gly Ser Asp His Cys Pro Ile Thr Leu Tyr Leu Ala Leu
   305 310 315
<210> 6
   <211> 760
   <212> PRT
   <213> Homo sapiens
<400> 6
   Met Lys Thr Trp Val Lys Ile Val Phe Gly Val Ala Thr Ser Ala Val
   1 5 10 15
   Leu Ala Leu Leu Val Met Cys Ile Val Leu Arg Pro Ser Arg Val His
   20 25 30
   Asn Ser Glu Glu Asn Thr Met Arg Ala Leu Thr Leu Lys Asp Ile Leu
   35 40 45
   Asn Gly Thr Phe Ser Tyr Lys Thr Phe Phe Pro Asn Trp Ile Ser Gly
   50 55 60
   Gln Glu Tyr Leu His Gln Ser Ala Asp Asn Asn Ile Val Leu Tyr Asn
   65 70 75 80
   Ile Glu Thr Gly Gln Ser Tyr Thr Ile Leu Ser Asn Arg Thr Met Lys
   85 90 95
   Ser Val Asn Ala Ser Asn Tyr Gly Leu Ser Pro Asp Arg Gln Phe Val
   100 105 110
   Tyr Leu Glu Ser Asp Tyr Ser Lys Leu Trp Arg Tyr Ser Tyr Thr Ala
   115 120 125
   Thr Tyr Tyr Ile Tyr Asp Leu Ser Asn Gly Glu Phe Val Arg Gly Asn
   130 135 140
   Glu Leu Pro Arg Pro Ile Gln Tyr Leu Cys Trp Ser Pro Val Gly Ser
   145 150 155 160
   Lys Leu Ala Tyr Val Tyr Gln Asn Asn Ile Tyr Leu Lys Gln Arg Pro
                   165                 170                 175
   Gly Asp Pro Pro Phe Gln Ile Thr Phe Asn Gly Arg Glu Asn Lys Ile
               180                 185                 190
   Phe Asn Gly Ile Pro Asp Trp Val Tyr Glu Glu Glu Met Leu Ala Thr
           195                 200                 205
   Lys Tyr Ala Leu Trp Trp Ser Pro Asn Gly Lys Phe Leu Ala Tyr Ala
       210                 215                 220
   Glu Phe Asn Asp Thr Asp Ile Pro Val Ile Ala Tyr Ser Tyr Tyr Gly
   225                 230                 235                 240
   Asp Glu Gln Tyr Pro Arg Thr Ile Asn Ile Pro Tyr Pro Lys Ala Gly
                   245                 250                 255
   Ala Lys Asn Pro Val Val Arg Ile Phe Ile Ile Asp Thr Thr Tyr Pro
               260                 265                 270
   Ala Tyr Val Gly Pro Gln Glu Val Pro Val Pro Ala Met Ile Ala Ser
           275                 280                 285
   Ser Asp Tyr Tyr Phe Ser Trp Leu Thr Trp Val Thr Asp Glu Arg Val
       290                 295                 300
   Cys Leu Gln Trp Leu Lys Arg Val Gln Asn Val Ser Val Leu Ser Ile
   305                 310                 315                 320
   Cys Asp Phe Arg Glu Asp Trp Gln Thr Trp Asp Cys Pro Lys Thr Gln
                   325                 330                 335
   Glu His Ile Glu Glu Ser Arg Thr Gly Trp Ala Gly Gly Phe Phe Val
               340                 345                 350
   Ser Thr Pro Val Phe Ser Tyr Asp Ala Ile Ser Tyr Tyr Lys Ile Phe
           355                 360                 365
   Ser Asp Lys Asp Gly Tyr Lys His Ile His Tyr Ile Lys Asp Thr Val
       370                 375                 380
   Glu Asn Ala Ile Gln Ile Thr Ser Gly Lys Trp Glu Ala Ile Asn Ile
   385                 390                 395                 400
   Phe Arg Val Thr Gln Asp Ser Leu Phe Tyr Ser Ser Asn Glu Phe Glu
                   405                 410                 415
   Glu Tyr Pro Gly Arg Arg Asn Ile Tyr Arg Ile Ser Ile Gly Ser Tyr
               420                 425                 430
   Pro Pro Ser Lys Lys Cys Val Thr Cys His Leu Arg Lys Glu Arg Cys
           435                 440                 445
   Gln Tyr Tyr Thr Ala Ser Phe Ser Asp Tyr Ala Lys Tyr Tyr Ala Leu
       450                 455                 460
   Val Cys Tyr Gly Pro Gly Ile Pro Ile Ser Thr Leu His Asp Gly Arg
   465                 470                 475                 480
   Thr Asp Gln Glu Ile Lys Ile Leu Glu Glu Asn Lys Glu Leu Glu Asn
                   485                 490                 495
   Ala Leu Lys Asn Ile Gln Leu Pro Lys Glu Glu Ile Lys Lys Leu Glu
               500                 505                 510
   Val Asp Glu Ile Thr Leu Trp Tyr Lys Met Ile Leu Pro Pro Gln Phe
           515                 520                 525
   Asp Arg Ser Lys Lys Tyr Pro Leu Leu Ile Gln Val Tyr Gly Gly Pro
       530                 535                 540
   Cys Ser Gln Ser Val Arg Ser Val Phe Ala Val Asn Trp Ile Ser Tyr
   545                 550                 555                 560
   Leu Ala Ser Lys Glu Gly Met Val Ile Ala Leu Val Asp Gly Arg Gly
                   565                 570                 575
   Thr Ala Phe Gln Gly Asp Lys Leu Leu Tyr Ala Val Tyr Arg Lys Leu
               580                 585                 590
   Gly Val Tyr Glu Val Glu Asp Gln Ile Thr Ala Val Arg Lys Phe Ile
           595                 600                 605
   Glu Met Gly Phe Ile Asp Glu Lys Arg Ile Ala Ile Trp Gly Trp Ser
       610                 615                 620
   Tyr Gly Gly Tyr Val Ser Ser Leu Ala Leu Ala Ser Gly Thr Gly Leu
   625                 630                 635                 640
   Phe Lys Cys Gly Ile Ala Val Ala Pro Val Ser Ser Trp Glu Tyr Tyr
                   645                 650                 655
   Ala Ser Val Tyr Thr Glu Arg Phe Met Gly Leu Pro Thr Lys Asp Asp
   660 665 670
   Asn Leu Glu His Tyr Lys Asn Ser Thr Val Met Ala Arg Ala Glu Tyr
   675 680 685
   Phe Arg Asn Val Asp Tyr Leu Leu Ile His Gly Thr Ala Asp Asp Asn
   690 695 700
   Val His Phe Gln Asn Ser Ala Gln Ile Ala Lys Ala Leu Val Asn Ala
   705 710 715 720
   Gln Val Asp Phe Gln Ala Met Trp Tyr Ser Asp Gln Asn His Gly Leu
   725 730 735
   Ser Gly Leu Ser Thr Asn His Leu Tyr Thr His Met Thr His Phe Leu
   740 745 750
   Lys Gln Cys Phe Ser Leu Ser Asp
   755 760
<210> 7
   <211> 766
   <212> PRT
   <213> Homo sapiens
<400> 7
   Met Lys Thr Pro Trp Lys Val Leu Leu Gly Leu Leu Gly Ala Ala Ala
   1               5                   10                  15
   Leu Val Thr Ile Ile Thr Val Pro Val Val Leu Leu Asn Lys Gly Thr
               20                  25                  30
   Asp Asp Ala Thr Ala Asp Ser Arg Lys Thr Tyr Thr Leu Thr Asp Tyr
           35                  40                  45
   Leu Lys Asn Thr Tyr Arg Leu Lys Leu Tyr Ser Leu Arg Trp Ile Ser
       50                  55                  60
   Asp His Glu Tyr Leu Tyr Lys Gln Glu Asn Asn Ile Leu Val Phe Asn
   65                  70                  75                  80
   Ala Glu Tyr Gly Asn Ser Ser Val Phe Leu Glu Asn Ser Thr Phe Asp
                   85                  90                  95
   Glu Phe Gly His Ser Ile Asn Asp Tyr Ser Ile Ser Pro Asp Gly Gln
               100                 105                 110
   Phe Ile Leu Leu Glu Tyr Asn Tyr Val Lys Gln Trp Arg His Ser Tyr
   115 120 125
   Thr Ala Ser Tyr Asp Ile Tyr Asp Leu Asn Lys Arg Gln Leu Ile Thr
   130 135 140
   Glu Glu Arg Ile Pro Asn Asn Thr Gln Trp Val Thr Trp Ser Pro Val
   145 150 155 160
   Gly His Lys Leu Ala Tyr Val Trp Asn Asn Asp Ile Tyr Val Lys Ile
   165 170 175
   Glu Pro Asn Leu Pro Ser Tyr Arg Ile Thr Trp Thr Gly Lys Glu Asp
   180 185 190
   Ile Ile Tyr Asn Gly Ile Thr Asp Trp Val Tyr Glu Glu Glu Val Phe
   195 200 205
   Ser Ala Tyr Ser Ala Leu Trp Trp Ser Pro Asn Gly Thr Phe Leu Ala
   210 215 220
   Tyr Ala Gln Phe Asn Asp Thr Glu Val Pro Leu Ile Glu Tyr Ser Phe
   225 230 235 240
   Tyr Ser Asp Glu Ser Leu Gln Tyr Pro Lys Thr Val Arg Val Pro Tyr
   245 250 255
   Pro Lys Ala Gly Ala Val Asn Pro Thr Val Lys Phe Phe Val Val Asn
   260 265 270
   Thr Asp Ser Leu Ser Ser Val Thr Asn Ala Thr Ser Ile Gln Ile Thr
   275 280 285
   Ala Pro Ala Ser Met Leu Ile Gly Asp His Tyr Leu Cys Asp Val Thr
   290 295 300
   Trp Ala Thr Gln Glu Arg Ile Ser Leu Gln Trp Leu Arg Arg Ile Gln
   305 310 315 320
   Asn Tyr Ser Val Met Asp Ile Cys Asp Tyr Asp Glu Ser Ser Gly Arg
   325 330 335
   Trp Asn Cys Leu Val Ala Arg Gln His Ile Glu Met Ser Thr Thr Gly
   340 345 350
   Trp Val Gly Arg Phe Arg Pro Ser Glu Pro His Phe Thr Leu Asp Gly
   355 360 365
   Asn Ser Phe Tyr Lys Ile Ile Ser Asn Glu Glu Gly Tyr Arg His Ile
       370                 375                 380
   Cys Tyr Phe Gln Ile Asp Lys Lys Asp Cys Thr Phe Ile Thr Lys Gly
   385                 390                 395                 400
   Thr Trp Glu Val Ile Gly Ile Glu Ala Leu Thr Ser Asp Tyr Leu Tyr
                   405                 410                 415
   Tyr Ile Ser Asn Glu Tyr Lys Gly Met Pro Gly Gly Arg Asn Leu Tyr
               420                 425                 430
   Lys Ile Gln Leu Ser Asp Tyr Thr Lys Val Thr Cys Leu Ser Cys Glu
           435                 440                 445
   Leu Asn Pro Glu Arg Cys Gln Tyr Tyr Ser Val Ser Phe Ser Lys Glu
       450                 455                 460
   Ala Lys Tyr Tyr Gln Leu Arg Cys Ser Gly Pro Gly Leu Pro Leu Tyr
   465                 470                 475                 480
   Thr Leu His Ser Ser Val Asn Asp Lys Gly Leu Arg Val Leu Glu Asp
                   485                 490                 495
   Asn Ser Ala Leu Asp Lys Met Leu Gln Asn Val Gln Met Pro Ser Lys
               500                 505                 510
   Lys Leu Asp Phe Ile Ile Leu Asn Glu Thr Lys Phe Trp Tyr Gln Met
           515                 520                 525
   Ile Leu Pro Pro His Phe Asp Lys Ser Lys Lys Tyr Pro Leu Leu Leu
       530                 535                 540
   Asp Val Tyr Ala Gly Pro Cys Ser Gln Lys Ala Asp Thr Val Phe Arg
   545                 550                 555                 560
   Leu Asn Trp Ala Thr Tyr Leu Ala Ser Thr Glu Asn Ile Ile Val Ala
                   565                 570                 575
   Ser Phe Asp Gly Arg Gly Ser Gly Tyr Gln Gly Asp Lys Ile Met His
               580                 585                 590
   Ala Ile Asn Arg Arg Leu Gly Thr Phe Glu Val Glu Asp Gln Ile Glu
           595                 600                 605
   Ala Ala Arg Gln Phe Ser Lys Met Gly Phe Val Asp Asn Lys Arg Ile
       610                 615                 620
   Ala Ile Trp Gly Trp Ser Tyr Gly Gly Tyr Val Thr Ser Met Val Leu
   625                 630                 635                 640
   Gly Ser Gly Ser Gly Val Phe Lys Cys Gly Ile Ala Val Ala Pro Val
                   645                 650                 655
   Ser Arg Trp Glu Tyr Tyr Asp Ser Val Tyr Thr Glu Arg Tyr Met Gly
               660                 665                 670
   Leu Pro Thr Pro Glu Asp Asn Leu Asp His Tyr Arg Asn Ser Thr Val
           675                 680                 685
   Met Ser Arg Ala Glu Asn Phe Lys Gln Val Glu Tyr Leu Leu Ile His
       690                 695                 700
   Gly Thr Ala Asp Asp Asn Val His Phe Gln Gln Ser Ala Gln Ile Ser
   705                 710                 715                 720
   Lys Ala Leu Val Asp Val Gly Val Asp Phe Gln Ala Met Trp Tyr Thr
                   725                 730                 735
   Asp Glu Asp His Gly Ile Ala Ser Ser Thr Ala His Gln His Ile Tyr
               740                 745                 750
   Thr His Met Ser His Phe Ile Lys Gln Cys Phe Ser Leu Pro
           755                 760                 765
<210> 8
   <211> 100
   <212> DNA
   <213> Artificial Sequence
<220>
   <223> Forward primer LC56for-EcoRI
<400> 8
   acgtacgtga attcattaaa gaggagaaat taactatatg agaggatcgc atcaccatca        60
   ccatcacatt gaaggccgta ggaggatgtg ggccacgcag                             100
<210> 9
   <211> 40
   <212> DNA
   <213> Artificial Sequence
<220>
   <223> Reverse primer LC56rev-BamHI
<400> 9
   acgtacgtgg atcctcatta caaatcggtc cgtgcactgg 40
<210> 10
   <211> 14
   <212> PRT
   <213> Artificial Sequence
<220>
   <223> N-terminal peptide extension
<400> 10
   Met Arg Gly Ser His His His His His His Ile Glu Gly Arg
   1               5                   10